# EUROPEAN PATENT SPECIFICATION

(11) **EP 2 412 806 B1**
(45) Date of publication and mention of the grant of the patent: **08.01.2014**
(21) Application number: 10290424.0
(22) Date of filing: 28.07.2010
(51) Int. Cl.: C12N 15/09

(54) **Use of terminal deoxynucleotidyl transferase for mutagenic DNA repair to generate variability, at a determined position in DNA**
Verwendung von terminaler Desoxynucleotidyltransferase zur mutagenen DNA-Reparatur zur Erzeugung von Variabilität an einer bestimmten Position in DNA
Utilisation d'une désoxynucléotidyle transférase terminale pour la réparation d'ADN mutagène pour générer de la variabilité en une position déterminée d'ADN

(43) Date of publication of application: 01.02.2012
(73) Proprietor: Institut Pasteur, 75724 Paris Cédex 15 (FR); Commissariat à l'Énergie Atomique et aux Énergies Alternatives, 75015 Paris (FR); Centre National de la Recherche Scientifique, 75794 Paris Cedex 16 (FR)
(72) Inventor: Rougeon, François, 92310 Sèvres (FR); Azzouz-Boubakour, Imenne, 94000 Créteil (FR); Lopez, Bernard, 91190 Gif sur Yvette (FR); Bertrand, Pascale, 91190 Gif sur Yvette (FR)
(74) Representative: Desaix, Anne

(56) References cited:
- BOUBAKOUR-AZZOUZ ET AL: "Low joining efficiency and non-conservative repair of two distant double-strand breaks in mouse embryonic stem cells", DNA REPAIR, ELSEVIER, AMSTERDAM, NL, vol. 7, no. 2, 26 October 2007 (2007-10-26), pages 149-161, XP022408850, ISSN: 1568-7864, DOI: DOI:10.1016/J.DNAREP.2007.09.005
- ROMAIN FELIX ET AL: "Conferring a template-dependent polymerase activity to terminal deoxynucleotidyltransferase by mutations in the Loop1 region", NUCLEIC ACIDS RESEARCH, vol. 37, no. 14, August 2009 (2009-08), pages 4642-4656, XP002615643, ISSN: 0305-1048
- MAHANEY BRANDI L ET AL: "Repair of ionizing radiation-induced DNA double-strand breaks by non-homologous end-joining", BIOCHEMICAL JOURNAL, vol. 417, no. Part 3, February 2009 (2009-02), pages 639-650, XP002615644,
- PENG LIMING ET AL: "A method for assessing strand breaks in DNA", ANALYTICAL BIOCHEMISTRY, vol. 262, no. 1, 15 August 1998 (1998-08-15), pages 9-16, XP002615645, ISSN: 0003-2697

## Description

The invention relates to the use of deoxynucleotidyl transferase (TdT) for mutagenic DNA repair of double-strand breaks (DSB). In this respect, the invention especially concerns a method involving use of TdT for mutagenic DNA repair in a DNA present in a chromosomal context in eukaryotic cells. The invention therefore relates to the nucleic acids and cells obtained through implementation of the method disclosed herein. The invention accordingly enables generation of variability in DNA, as a result of mutagenic DNA repair.

The method of the invention, involving the use of TdT is especially designed for DNA repair in a nucleic acid, and therefore comprises at least one step enabling DNA double-strand breaks (DSB) to be performed in a locus in the nucleic acid.

The invention therefore concerns the use of the method of generating mutagenic DNA repair for the generation of collections of mutated nucleic acids or for the preparation of collections (populations) of cells which differ from each other by the sequence of the repaired junction(s) at the double-strand break site(s) in said DNA.

The method of the invention may be carried out on a targeted DNA or on a random DNA present in a chromosomal context (target DNA). For each of these candidate DNAs the double-strand break(s) may be performed as targeted DSB(s) or as random DSB(s).

The invention may be used for the generation of mutations in nucleic acid(s), wherein said mutations are suitable to modify, elicit, restore, improve, lower or abolish properties (i.e., structural or functional features) of said nucleic acid, and/or properties of adjacent nucleic acid(s) and/or properties of functionally related nucleic acid(s), and/or properties of expression product(s) of such nucleic acid(s). The invention therefore provides means to assess genetic regulation of nucleic acids in a chromosomal context, or to assess structure-function relationships in nucleic acids. The invention also provides means enabling the generation of new products, e.g., new nucleic acids or expression products thereof.

The method of the invention allows the production of mutated nucleic acid(s), especially mutated polynucleotide(s) in a chromosomal context. The open reading frame of a mutated polynucleotide (if the polynucleotide comprises or is a coding sequence) may be either modified or not modified with respect to the original nucleic acid sequence. The mutated nucleid acids may also contain one or more coding sequence(s) corresponding to one or several genes. In this last case gene functions may be either modified or not modified with respect to the original function of the original non-mutated sequence, including possibly modified in a way resulting in an abolished function.

The method of the invention thus also allows the production of collections of recombinant clones, in which each clone comprises at least a polynucleotide of interest having undergone mutagenic repair.

The invention also provides modified cells, in particular a population of cells, especially cells having modified properties when they comprise the mutated nucleic acid, e.g., but not exclusively, cells having a modified phenotype. The cells are eukaryotic cells, either cells originating from unicellular organisms or cells originating from multicellular organisms. These cells may include yeast cells, fungus cells, and in particular cells originating from Vertebrates, especially mammalian cells, including in particular human cells, murine, especially mice cells, or cells originating from birds (e.g., chicken) or fish, or cells originating from plants.

The method of the invention also allows the determination of occurrence(s) of generation of DSB(s) in a cell, or in a population of cells, wherein said determination encompasses evaluation of the junctional variability generated in said cell. In a particular embodiment, such DSB(s) can be generated by at least a nuclease, especially a meganuclease.

The invention further concerns the use of Terminal deoxynucleotidyl transferase (TdT) as a marker of DSB events, wherein a DSB is repaired in a way generating junctional variability at the locus of said DSB.

The present invention may be used in various fields including in or for medical applications, biotechnology applications, food industry, agrobusiness or in or for applications in plant technologies.

Terminal deoxynucleotidyl transferase (TdT) is a polymerase that by adding non-templated nucleotides to V(D)J recombination junctions increases the repertoire of antigen receptors. The inventors have made the hypothesis that, although naturally lymphocyte-specific, expression of TdT may be of interest if induced in other cell types and if such expression could be mutagenic and thereby add nucleotides to junctions derived from DNA double-strand breaks (DSB) in a context different from V(D)J recombination junctions.

When generated *in vivo* in organisms, DNA double-strand breaks (DSBs) should be repaired as accurately as possible to avoid mutations, for example in oncogenes or tumor suppressor genes that would lead to cancer initiation. Non-homologous end-joining (NHEJ) is a major repair mechanism (Hasty, 2008) (Weterings and Chen, 2008). According to this mechanism, DNA ends are recognized by the Ku heterodimer and the catalytic subunit DNA-PK_{CS}. These proteins then act as a scaffold for the stable recruitment of the XRCC4-ligaseIV complex that joins DNA ends. The inventors have recently shown the existence of an alternative pathway to this so-called canonical NHEJ pathway, when proteins Ku80 or XRCC4 are deficient (Guirouilh-Barbat et al., 2004) (Guirouilh-Barbat et al., 2007). However, this has a cost, since this alternative pathway is highly mutagenic. According to the alternative NHEJ pathway (A-NHEJ formerly also referred to as NHEJ-alt), the resection of the single-strand ends that cannot be protected from exonucleolytic activity in the absence of Ku80 and the use of internal microhomologies distant from the initial point breaks lead to extended deletions at the junction (Guirouilh-Barbat et al., 2004; Guirouilh-Barbat et al., 2007).

In contrast with general DSB repair, in cells of the immunological system, the repair of V(D)J recombination generates genetic variability, thus favouring the diversity of the immune repertoire. During V(D)J recombination, a first level of diversity is created by the rearrangement of variable (V), diversity (D) and joining (J) Ig and TCR gene segments, generating then around 10⁹ distinct antibody molecules. The lymphoid-specific components of the recombination machinery, RAG1 and RAG2, initiate the process by generating two DNA double-strand break (DSBs) at recombination signal sequences that are adjacent to the V, D and J coding segments (Jung and Alt, 2004). Subsequently, the joining of the DNA ends is processed by the nonlymphoid-restricted components of canonical NHEJ: Ku70, Ku80, DNA-PKcs, XRCC4, ligaseIV, and Artemis (Rooney et al., 2004) (Lieber et al., 2004). To this combinatorial diversity is added a junctional diversity. This second level of diversity is characterized by unfaithful repair of coding joints that typically exhibit both loss of nucleotides and addition of extra-nucleotides. Two distinct mechanisms operate to add extra-nucleotides. P (palindromic) nucleotides result from hairpin structures in cleavage intermediates (Lewis, 1994a). N (non-templated) nucleotides are added by the lymphocyte-specific terminal deoxynucleotidyl transferase (TdT) (Desiderio et al., 1984) (Benedict et al., 2000).

TdT is a polymerase that belongs to the Pol X family polymerase (Nick McElhinny and Ramsden, 2004) ; it adds nucleotides randomly to 3' ends of nucleotide sequences (Kato et al., 1967) (Bollum, 1978). The inventors first reported the expression through alternative splicing of two isoforms of TdT in the mouse (Doyen et al., 1993), the only species with two such isoforms. Unlike murine TdT short (TdTS) isoform, murine TdT long (TdTL) isoform, which has a 20-aa (amino acids) insertion, cannot add N regions to V(D)J junctions and tends to remain in the cytoplasm of transfected cells, where it is rapidly degraded (Bentolila et al., 1995) (Doyen et al., 2004). *In vitro,* TdT can catalyse up to 1 kb nucleotides addition to any DNA end containing a 3'-OH whereas *in vivo,* only a few nucleotides (on average, 2-5) are added to coding ends with a marked bias toward dGTP and dCTP additions (Chang and Bollum, 1986) (Robbins et al., 1987) (Robbins and Coleman, 1988). N nucleotides are present at more than 70% of junctions at TCR and Ig loci (Shimizu and Yamagishi, 1992) (Iwasato and Yamagishi, 1992). In TdT-deficient mice N nucleotides additions at coding joints are very rare (Komori et al., 1993) whereas in mice with a constitutive expression of TdT N additions are observed even in light chains (Bentolila et al., 1997).

Despite extensive studies on TdT gene regulation (Cherrier et al., 2008), protein structure (Delarue et al., 2002) and enzymatic activity (Boule et al., 2001), the partners of TdT for N addition still remain to be defined. *In vitro* studies suggested that TdT does not need any other factor to add N nucleotides (Robbins and Coleman, 1988). However, the absence of N additions in the rare coding joints of Ku80 deficient mice suggested that this NHEJ factor is necessary for TdT recruitment to the nucleus, to DNA ends or for its activation (Bogue et al., 1997). In 2001, Purugganan *et al.* have shown an absence of N additions with a V(D)J episomal substrate in a Ku-deficient CHO cell line where TdT is ectopically expressed. The correct folding of the enzyme to the nucleus and its intact enzymatic activity in a Ku-deficient background lead the authors to suggest that TdT does not add nucleotide by simple collision but rather *via* a Ku-dependent mechanism. In contrast, a recent study has shown by using a non V(D)J episomic plasmid substrate a Ku-independent N addition process by TdT and that the additions are abnormally long in the absence of Ku80 (11-27 nt in comparison to 1-5 nt in wild-type cells), suggesting a negative control of TdT activity by Ku80. Another possibility is that the junctions generated in the absence of Ku80 may be formed by an alternative NHEJ pathway, like the one the inventors previously described (Guirouilh-Barbat et al., 2007), which would affect the activity of TdT. Indeed, an alternative NHEJ pathway has also been suggested to take place in the V(D)J context (Yan et al., Nature, 2007 ; Soulas-Spreague et al., J Ex Med, 2007 ; Comeo et al., 2007). Moreover, the use of microhomologies by the V(D)J recombination machinery has been detected in several studies (Gerstein and Lieber, 1993) (Corneo et al., 2007). XRCC4 is a protein essential in the classical/canonical end joigning (Guirouilh-Barbat et al., 2007) but is not involved in the alternative end joigning pathway. Probably because XRCC4-deficient mice are not viable (Gao et al., 2000) and the alternative end-joining pathway has been only recently described, very few studies dealt with the partnership between TdT and XRCC4. In addition, until now, data are very confusing with regard to the recruitment of TdT by Ku80 to DSBs. Ku and TdT from cell extracts do not always co-immunoprecipitate (Mahajan et al., 1999) (Repasky et al., 2004). Biochemical studies that show a binding of TdT to oligonucleotides in the absence of Ku and an association between TdT and XRCC4 urge on studying the involvement of XRCC4 in N addition by TdT (Ma et al., 2004; Mahajan et al., 2002).

The study of Sandor et al (Sandor et al., 2004) was performed, to analyze, on plasmids substrates, the dependency on the expression of Ku protein, of N additions by TdT to non-V(D)J DSBs. If, according to *in vitro* studies, no known mechanism would prevent the addition of N nucleotides to random DSBs when TdT is expressed, Sandor et al.'s conclusions were that N-additions by TdT in the absence of Ku were possible but resulted in frequently abnormally long additions. Sandor et al. remained silent about the possible implication of XRCC4 in these N-additions, and did not measure the impact of Ku in a chromosomal context.

Incidentally, abnormal N additions have been observed in Ig light chain genes (Bentolila et al., 1997) and in other loci where DSBs were not intermediates in V(D)J recombination (Sale and Neuberger, 1998) (Murray et al., 2006).

However, TdT expression is subjected to a very tight spatio-temporal control. Such regulated expression would help to prevent TdT from acting at non-V(D)J DSBs, which would be highly mutagenic in the context of general DSB repair.

In order to determine whether the use of terminal deoxynucleotidyl (TdT) would provide an interesting means for the generation of mutations at sites of DSB repair, the inventors have designed experiments which enable to assess how TdT could perform mutagenic repair of DSBs in eukaryotic cells, on nucleic acids present in a chromosomal context. Using a chromosomal substrate, the inventors have shown that TdT efficiently adds a limited number of N nucleotides (Non-templated nucleotides) which addition possibly does not interfere with the function of the nucleic acid thus modified at the junction of DSBs and that this process is Ku and XRCC4 dependent, i.e., makes use of the known canonical Non Homologous End-Joining pathway (NHEJ). By contrast, the alternative NHEJ pathway (A-NHEJ formerly also referred to as NHEJ-alt) is considered as a generator of genetic instability.

The results which have been obtained allow the design of a new method of generating variability in the nucleotide sequence of nucleic acids or polynucleotides in particular target nucleic acid or target polynucleotides.

The new method of generating variability in the nucleotide sequence of nucleic acids or polynucleotides of the invention can be applied *ex vivo* (in particular *in vitro),* for example, but not exclusively, in conditions involving cultured cells.

In a particular embodiment, the new method of the invention can be applied *in vivo,* for example on animals, especially non-human animals.

Specifically, the method of the invention uses the canonical NHEJ pathway to efficiently generate mutants, thereby providing a balance between preservation of the host genome integrity and generation of adequately variable and diverse mutants.

The invention thus relates to a method of generating junctional variability in the nucleotide sequence of a polynucleotide of interest present in an intrachromosomal (also designated chromosomal) substrate or context in a eukaryotic cell competent for canonical Non Homologous End Joining pathway (NHEJ) repair, comprising the steps of:
a) generating a double-strand break (DSB) in the DNA sequence of said polynucleotide, thereby providing broken ends in said polynucleotide in said eukaryotic cells,
b) providing the polymerase Terminal Deoxynucleotidyl Transferase (TdT) as a functional protein in the cells resulting from step a), in conditions enabling said TdT to add Non-templated nucleotides (N nucleotides) to the 3' ends of said broken ends before ligation of said ends through canonical Non Homologous End Joining pathway (NHEJ) thereby allowing a mutagenic repair to take place at the DSB site.

In a particular embodiment, the method of the invention is performed *ex vivo* (or *in vitro*) and generates junctional variability in the nucleotide sequence of a polynucleotide of interest present in an intrachromosomal (also designated chromosomal) substrate or context in a eukaryotic cell which is competent for canonical Non Homologous End Joining pathway (NHEJ) repair.

In a particular embodiment, the method of the invention allows the generation of conservative junctional variability in the nucleotide sequence of a polynucleotide present in an intrachromosomal substrate (i.e., in a chromosomal context) in a eukaryotic cell which is competent for the canonical NHEJ pathway.

In particular embodiments, the method of the invention is carried out in such a way that the double-strand break (DSB) is generated as (i) a targeted DSB in the DNA sequence of either a target polynucleotide or a random polynucleotide or as (ii) a random DSB in the DNA sequence of either a target polynucleotide or a random polynucleotide DNA sequence.

According to a particular embodiment, the eukaryotic cell on which the method of the invention is performed does not naturally express a functional Terminal Deoxynucleotidyl Transferase (TdT). In such an embodiment, the cells are non lymphoid cells.

The TDT which is involved to carry out step (b) of the method of the invention is chosen for its capacity to be active in the cells where DSB(s) is (are) generated. The TDT may in particular be a TdT known to be expressed in eukaryotic cells, in particular naturally expressed in murine cells or in human cells (human TdT). For illustration purpose, human TdT is the protein having the sequence disclosed in Genebank AAA36726.1 (disclosed as SEQ ID NO: 110).

When TdT exists as a short and as a long form of the protein, the invention especially relates to the use of the short form. Characteristics relating to TdT are also herein disclosed by reference to the data available in the state of the art, mentioned above in the present description.

When the method of the invention is performed *ex vivo,* it involves steps which are carried out on cells, outside of the body or organism from which said cells are possibly obtained or from which they originate. Accordingly, said cells are maintained, cultured or propagated outside of the body or organism. The expression *ex vivo* accordingly includes *in vitro.*

The method of the invention involving the use of TdT is carried out on eukaryotic cells, whether these cells originate from a unicellular organism or from a multicellular especially complex organism, including cells originating from yeast or fungi, or including cells originating from animals, in particular from Vertebrates or advantageously from mammalian and especially human, or murine, especially mice cells or bird cells such as chicken cells or fish cells, or cells originating from plants. Said cells are competent or are rendered competent for canonical Non-homologous end-joining pathway (NHEJ) either because they naturally express the compounds necessary for said NHEJ pathway or because they have been rendered suitable for said NHEJ pathway, as a result of modification such as expression complementation of the necessary components.

In other words, eukaryotic cells competent for canonical NHEJ repair according to the invention are cells which provide conditions that enable TdT polymerase to be active in particular on a Ku and XRCC4-dependent manner, in line with what has been observed for TdT activity on V(D)J intrachromosomal recombination junctions in the process of increasing the repertoire of antigen receptors for immature immunological B or T cells. Accordingly, eukaryotic cells used to perform the invention are not XRCC4-deficient cells and are not Ku-deficient cells, since the method of the invention requires that the Ku-XRCC-4 pathway for canonical NHEJ is functional. Cells which would be deficient for enzymes of the canonical NHEJ pathway may be complemented to become competent for said pathway. Complementation for the deficient protein can be achieved through expression complementation, as mentioned above, or through the punctual supplementation of the deficient protein.

The method of the invention requires TdT to be provided as a functional protein. TdT can be provided by supplying the functional protein, or provided by bringing the TdT coding sequence into the cell, especially into the cell's genome in conditions enabling its expression, and/or provided by inducing its expression in a cell where the TdT coding sequence is naturally present, or was brought on purpose. In a particular embodiment where the cells do not naturally express TdT, such as non lymphoid cells, TdT is provided as an exogenous protein. TdT supply includes the particular case where TdT expression is induced after insertion of a TdT coding sequence into the cell's genome.

For the purpose of the invention, the main steps and components for the canonical NHEJ to be functional are disclosed in the introductory section of the present application in accordance with what has been described in the literature in this field and mainly involves a KU heterodimer (involving KU70 and KU80), the catalytic subunit DNA-PK_{CS} Artemis enzyme, and the recruitment of the XRCC4-XLF-ligase IV complex (also called XRCC4-Cemunnos-ligase IV complex) that enables DNA ends to join. It is specified that in principle all eukaryotic cells are competent for the canonical NHEJ pathway. In particular for the purpose of the invention cells may be assessed for their ability to express Ku and XRCC4, if such assessment is needed.

In the method of the invention, a polynucleotide of interest undergoes modifications, and especially is targeted for modifications, at the level of broken ends resulting from DNA double-strand break(s), especially targeted DSB, and said modifications occur when said polynucleotide of interest is contained in a chromosomal context i.e., is present in a chromosomal substrate.

According to the invention, a polynucleotide in which a DSB is generated is defined as a "polynucleotide of interest". According to a particular embodiment, a polynucleotide of interest can be a targeted polynucleotide. Targeting in this respect may rely on criteria such as location into the genome, functional parameters of the target DNA, which are known or are to be identified, involvement in phenotypic traits, or structural parameters of the DNA. Targeting may take into consideration possible functional or structural relationship among multiple DNA. According to another particular embodiment, a polynucleotide of interest can be a random polynucleotide. A random polynucleotide is a polynucleotide which is not selected or targeted under predefined criteria for the step of generation DSB. A polynucleotide of interest can be a nucleic acid naturally present in a chromosome of the eukaryotic cell wherein the method of the invention is implemented, or can be a derivative or variant of such naturally occurring nucleic acid. Alternatively, in another embodiment of the invention, the said polynucleotide of interest is a nucleic acid which is heterologous with respect to the chromosomal nucleic acid of the eukaryotic cells wherein the invention is carried out. The expression "heterologous" means that said nucleic acid is originating from a different cell or organism than the cell type which is used to perform the invention, or is a non-naturally occurring nucleic acid such as a chimeric or an artificial nucleic acid. Such heterologous polynucleotide may be inserted in the genome of the cell.

The polynucleotide of interest which comprises, either naturally or by insertion, the cleavage site where the DSB is generated, may be a fragment of a larger nucleic acid; Such a fragment has advantageously more than 20 nucleotides and in particular has more than 100 nucleotides, especially more than 200.

The polynucleotide of interest may have been inserted and integrated into the chromosomal DNA of said eukaryotic cells, either randomly or alternatively in a targeted manner, as a result of a particular step performed before carrying out the invention. Such a step encompasses for example infection, transfection or transduction of the eukaryotic cells with the polynucleotide of interest using an appropriate vector such as a plasmid or a viral vector, especially a lentiviral vector or a protein vector.

Alternatively, the polynucleotide of interest may have been inserted into the chromosomal substrate through the action of an agent or of an organism, such as a pathogenic one, including a virus, a bacterium or a parasite. It may for example be present into the chromosomal substrate of the cell as a result of infection of the cell through a foreign agent especially a pathogenic agent or through infection by an organism, or as a result of the transformation of the cell following the infection of the organism from which it may originate.

The polynucleotide of interest can be in its native form, or it may have undergone modifications with respect to a reference wild-type form if any, especially when it is a polynucleotide which is inserted and integrated in the chromosomes of the cell. The modifications may be carried out prior to or after the insertion into the cell or as a result of recombination into the cell genome.

The polynucleotide of interest of the invention, either targeted or randomly considered (random polynucleotide), may be a nucleic acid of a gene or of a gene fragment, including an exon, an intron, an expression regulatory sequence such as a promoter, a coding sequence, a non coding sequence. It may be a nucleic acid of eukaryotic origin. It may be a nucleic acid, especially of prokaryotic origin, originating from a pathogenic organism, such as a viral or bacterial or parasite nucleic acid, including a protein coding sequence. It may be a nucleic acid of prokaryotic origin, originating from a non-pathogenic organism.

The polynucleotide of interest of the invention, either targeted or randomly considered (random polynucleotide), may be present as a unique sequence in the chromosomal substrate of the cell or rather may be present as multiple sequence copies, either contiguous in the chromosome or spread on the chromosome and/or on different chromosomes. Different polynucleotides, i.e., polynucleotides having different nucleotide sequences, present in the chromosomal substrate of the cell may be subject to the double-strand break.

According to a first step of the method of the invention, a DSB is generated in a targeted way in the DNA sequence of the polynucleotide, either a targeted polynucleotide or a random polynucleotide, which means that a specific locus of the polynucleotide is the target of the break in the eukaryotic cell.

The specific locus or site used as the target for the double-strand break can be naturally present in the DNA sequence of the polynucleotide, either a targeted polynucleotide or a random polynucleotide, or can be added or designed as a result of insertion(s) and/or mutation(s) in the sequence of said polynucleotide. Double-strand break sites are usually present as nucleotide sequences of a sufficient length to be considered as highly rare sequences. Preferably they are designed as unique sequences within the context of the chromosomal DNA of the cell of interest, meaning that they can be regarded as basically found only into the polynucleotide of interest.

In another embodiment of the invention, the site for the DSB is not a unique site, i.e., there can be multiple sites in the polynucleotide.

In a particular embodiment of the invention, sequences forming the recognition site for the DSB have 10 or more than 10bp, especially 12 or 15 or more than 12 or 15 nucleotides and for example from 12 (or 15) to 20, 22, 25, 30, 40 or 60bp, especially from 10 (or 12, or 15) to 60 or any length within these ranges.

Double-strand break site for the purpose of the invention may be unique in the polynucleotide of interest (giving rise to a single DSB event) or may be multiple (giving rise to multiple DSB events). Different DSB sites may be introduced into the same or into various copies of the same polynucleotide of interest or in different polynucleotides to obtain the polynucleotides of the invention, either targeted or random polynucleotides, as candidate for the DSB.

The DSB sites are especially suitable for the generation of DSB(s) as a result of the action of a nuclease, in particular a meganuclease. Alternative means to generate DSB(s) are however available and are illustrated hereafter.

In a second step of the method of the invention, the TdT, which has been provided to said cells as a functional protein, including when its expression has been induced, enables the broken ends, especially obtained as 3' overhang ends or as 3' blunt ends in said broken polynucleotide to be repaired through canonical NHEJ pathway, with either the addition of Non-templated nucleotides (N) or both the deletion of nucleotides contained at the end(s) of said broken ends and the addition of N nucleotides (N-nt) at these ends.

"Deletion at the 3'end" means deletion of nucleotide(s) at the extremity of the 3' end or in the immediate vicinity of said extremity, i.e., usually in a sequence of 1 to 10 nucleotides starting from said extremity, or in a window which extends over said immediate vicinity, as disclosed in the present application.

Accordingly, when a 3' overhang or a blunt end is obtained after the double-strand break has occurred, some nucleotides at said 3' overhang, or at said blunt end, may be deleted from the original polynucleotide sequence, before the addition of Non-templated nucleotides at the same 3' overhang or blunt end.

After said TdT has thus modified or mutated the 3' overhang(s) or blunt ends of the broken ends, the subsequent steps of the canonical NHEJ pathway enable the ligation of the broken ends through the added N nucleotides which act as template for hybridization of the 3' overhang of said ends. The repaired DNA which results from this process is mutagenic, meaning that it is modified following N-nt additions and optionally, deletions of some nucleotides as disclosed above, at the broken ends prior to said addition. Thus the process of the invention enables generation of randomly mutated polynucleotides in a chromosomal context.

In line with canonical NHEJ pathway, the number of deleted nucleotides at the 3' ends of the broken ends, is usually limited, and especially within the range of 0 to 60, preferably 0 to 15, or 0 to 10, in particular 1 or 2, 3, 4 or 5 nucleotide deletions. In particular, as illustrated in the examples, the number of deleted nucleotides can be of 1, 5, 6, 9, 15, 20, 22, 25, 26, or 30 deleted nucleotides with respect to the original polynucleotide (i.e. the polynucleotide sequence including the site for the DSB).

The number of N nucleotide(s) which is(are) added to the 3' ends of the broken ends resulting from the DSB is usually comprised within the range of 1 to 15, especially the range of 1 or 2, to 10, or from 1 or 2 to 6, in particular the range of 1 to 5, and in particular is 1, 2, 3, 4, 5, 6, 7, 8, 9, 10 nucleotides.

According to a particular embodiment of the invention, junctional variability that is obtained at the broken end(s) generated by the DSB, after said broken ends have been repaired, involves generally 1 to 10 nucleotides either deleted from or added to or deleted and added in the polynucleotide where said DSB is generated.

In a particular embodiment, the number of added nucleotide(s) is not primarily restricted to the number of deleted nucleotide(s) and vice versa. Accordingly, any combination between the above proposed numbers of nucleotide(s) deletion and proposed number of nucleotide(s) addition is within the frame of the invention to achieve the junctional variability. Illustration of said combined deletion and addition is given in the examples, and for example encompasses, for illustration purpose, the following combinations: any number among 0 to 55 deletions with either of 1, 2, 3, 4, 5, 6 and up to 9 additions.

In a particular embodiment of the invention the method enables a conservative addition of N nucleotides at the 3'- and/or 5'- ends of the broken junctions, meaning that there is a numerical relation between the number of deleted nucleotides and the number of added nucleotides, so that these numbers are identical or are balanced in such a way that the difference between the smallest with respect to the largest number (between addition and deletion) is not more than 50% calculated from the largest. As an example, these numbers of added and respectively deleted nucleotides are in a range as follows: for 1 added or respectively 1 deleted nucleotide, 0, 1 or 2 nucleotide(s) is(are) deleted or respectively added. In a particular embodiment of the invention, the amount of respectively deleted and added nucleotides at the level of the broken junctions is approximately of the same order. However, the balance between the amount of deleted and added nucleotides can be in favour of an overall addition of nucleotides at the level of the repaired broken junction.

In a particular embodiment, the amount of deleted and added nucleotides is in favour of an overall addition of nucleotides at the level of the repaired broken junction. More specifically, the amount of overall added nucleotides after repair is generally of about 1 to 10 nucleotides, especially 1 to 6 or 1 to 5.

In a particular embodiment, this amount of deleted and added nucleotides at the level of the repaired broken junction is determined in a so-called window constituted by a sequence of about 10 to 60 nucleotides, and generally about 50 nucleotides around the repaired broken junction resulting from the cleavage at the level of the DSB. This so-called window may be centered on the DSB cleavage site, or may be asymmetrically located around the DSB cleavage site. For example, the window can spread over about 20 nucleotides beginning from one end of the broken junction and about 30 nucleotides beginning from the other end of the broken junction.The present invention thus relates to a method enabling the occurrence of a limited number of mutations, within the above disclosed ranges, in the polynucleotide sequence as a consequence of the repair of DSBs, which mutations can be detected at the level of the ends of the broken junction for example in said so-called windows. As a consequence of the limited overall mutations according to the invention, the junctional variability generated in the nucleotide sequence of a polynucleotide of interest is defined as conservative.

In a particular embodiment, the added N nucleotides are contiguous at the generated broken ends and are provided at the extremity of the 3' or 5' end of the broken ends.

The N nucleotides are especially any nucleotide among A, T, C and G randomly added by the mutagenic repair process of the invention.

In accordance with embodiments disclosed herein, the method of the invention provides mutated polynucleotide(s) in a chromosomal context whose open reading frame (if the polynucleotide comprises or is a coding sequence) may be either modified or not modified and as a consequence whose encoded polypeptide is expressed or is not expressed anymore or, if expressed, may have kept its functional properties despite a modified sequence size or a similar size. Alternatively, the expressed mutated polypeptide, with or without a modified sequence size, may have a modified function with respect to the original function of the polypeptide encoded by the original polynucleotide including an abolished function.

The invention also concerns the generation of collection of clones and concerns the generated clones or polynucleotides having junctional variability after DSBs.

When a collection of cells is used to perform the invention, the mutagenic repair method of the invention gives rise to cells globally different in their genotype, because they contain different mutated polynucleotide(s) with different numbers of added nucleotides, and with or without different numbers of deleted nucleotides, at the level of the junctions resulting from repaired broken ends or in the so-called window of sequence. In a particular embodiment, the collection of cells obtained when performing the method of the invention or a subgroup in said collection may harbour modified phenotypic features.

The DSB in the polynucleotide in a chromosomal context may be obtained as a result of a direct chemical break, physical break or enzymatic break. Accordingly, the DSB may be generated by using a chemical reagent, a physical reagent, an enzyme or a combination thereof. The DSB in the polynucleotide in a chromosomal context may also be obtained indirectly as the result of the inhibition of DNA metabolism functions leading to chemical break(s), physical break(s) or enzymatic break(s), or a combination thereof.

Inhibition of DNA metabolism functions may be obtained through DNA replication-blocking, using for example agents interfering with DNA replication such as cis-platin, mitomicin C, psoralens and/or UV-A irradiation, or through nucleotides stock depletion using hydroxycarbamide or hydroxyurea, or through inhibition of proteins involved in DNA replication, or through inhibition of DNA replication, i.e. by agents such as aphidicolin, or a combination thereof. One may also inhibit genes coding for proteins involved in DNA replication, or use topoisomerase inhibitors.

Chemical breaks may be directly generated using for example agents such as EMDS, MMS (Methyl Methane Sulphate), or catalytic DNA such as TFO (Triplex Forming Oligonucleotide);

Physical breaks may also be generated using for example radiations such as y radiations; one may also use radiomimetic agents such as bleomycin or neacarcinostatin to obtain DSBs in the polynucleotide in a chromosomal substrate/context.

Enzymatic breaks may be generated using nucleases. A nuclease may be expressed endogenously in a host cell having recourse to different biological methods, such as co-transfection of a DNA molecule coding for said nuclease, or transfer of a RNAm molecule coding for said nuclease into the cell of interest, or injection or transfer of the protein corresponding to the nuclease directly into the cell of interest. It may alternatively be supplied as an active protein.

In a particular embodiment of the invention, DSBs are achieved through a method generating non-random breaks.

In a particular embodiment of the invention, the targeted DSB is generated by cleavage of the polynucleotide with a nuclease, especially a meganuclease, in particular a meganuclease chosen among Homing Endonucleases (HEs), artificial endonucleases such a Zinc Finger Nucleases, and engineered endonucleases. The cleavage is especially obtained at the DSB site which is a recognition site for the meganuclease or adjacent to said DSB site.

Meganucleases used in the process of the invention are sequence specific endonucleases which recognize large targets in nucleic acid sequences, especially targets having more than 10, in particular 12, 15 or more nucleotide bases, for example up to 20, 22, 25, 30, 40, 60 nucleotides and especially any length within the range of 10 to 60 nucleotides (Chevalier et al., 2001). Within the broad definition of meganucleases, the invention particularly relates to meganucleases which are called Homing Endonucleases (HEs) by reference to the homing process and engineered Homing Endonucleases such as endonucleases with enhanced specificity, modified targeting properties, modified binding specificity, lower possible toxicity in cells, enhanced cleavage efficacy in cells, or to custom-designed endonucleases.

A preferred endonuclease to carry out the invention is Homing Endonuclease I-Scel which has been extensively disclosed in processes of enhancing homologous gene targeting and for which genes and corresponding proteins are accessible in data bases. I-Scel is a HE which requires a recognition site of 18 base pairs (I-Scel recognition site) in order to cleave DNA to produce a DNA double-strand break. It has been disclosed that such a 18-base pair site does not naturally exist in most of mammalian genomes. Accordingly, when I-Scel is intended for use in order to induce DNA double-strand break in a targeted sequence of a mammalian genome, said genome must undergo some modifications in order to contain a recognition site for I-Scel, e.g., must be modified by the introduction of a I-Scel recognition site either randomly or at a determined locus (target site for the DSB) in the genome. Other enzymes can be used instead of I-Scel in the method of the invention. They include especially Homing Endonucleases suitable for use in the present invention such as:

| Endonuclease | Encoded by | Reference |
|---|---|---|
| I-*Sce*II | (Sc cox1-4 intron) | (Sargueil et al., NAR (1990) 18, 5659-5665) |
| I-*Sce*III | Sc cox1-3 intron | (Sargueil et al., MGG (1991) 225, 340-341) |
| I-*Sce*IV | Sc cox1-5a intron | (Seraphin et al., (1992) |
| I-*Ceu*I | Ce LSU-5 intron | (Marshall, Lemieux Gene (1991) 104, 241-245) |
| I-*Tev*I | T4 td-1 intron | (Chu et al., PNAS (1990) 87, 3574-3578 ) |
| I-*Tev*II | T4 sunY intron | (Bell-Pedersen et al., NAR (1990) 18, 3763-3770) |
| I-*Tev*III | RB3 nrdB-1 intron | (Eddy, Gold, Genes Dev. (1991) 5, 1032-1041) |
| HO | HO yeast gene | (Nickoloff et al., (1990) 10,1174-1179) |
| Endo *Sce*l | FR3 yeast mito. gene | (Kawasaki et al., JBC (1991) 266, 5342-5347) |
| I-Cre | UniProtKB/Swiss-Prot P05725 (database version number 2010_05/2010_05 released on April 20, 2010 - Entry version 74) | |
| Msol | UniProtKB/Swiss-Prot P53604 (database version number 2010_08 / 2010_08 released on July 13, 2010 - Entry version 66) | |
| I-Dmol | UniProtKB/Swiss-Prot P21505 (database version number 2010_05/2010_05 released on April 20, 2010 - Entry version 55) | |

Preferably endonucleases which directly allow a cleavage resulting in 3' protruding ends or blunt ends are used. If non blunt ends are obtained they may be modified to become accessible.

Other endonucleases, including Zinc Finger Nucleases, or engineered nucleases derived therefrom can be used provided they are suitable to target a specific sequence or motif in the target polynucleotide of the invention. Such engineered endonucleases that may be adapted to target sequences present in chromosome(s) which become their recognition site(s) have been disclosed as an example, in the publication of Pâques F et al (2007).

Artificial and custom-designed meganucleases might include type II restriction endonucleases, highly specific endonucleases such as an endonuclease wherein several recognition domains of homing endonucleases are fused, e.g.. domains of homing endonucleases I-Dmo I and I-Cre I, or meganucleases resulting from a fusion between nucleic acids and chemical compounds, in which DNA binding and specificity rely on an oligonucleotide and cleavage on a chemical compound tethered to the oligonucleotide. In this last case, the chemical compounds can have an endogenous cleavage activity, or cleave when complexed with other agents, such as topoisomerases.

The method of the invention is designed in a way that allows the addition by TdT of N nucleotides to the 3' ends of broken junctions resulting from the occurrence of the double-strand break. Accordingly, the DSB event, especially resulting from the use of a nuclease, especially a meganuclease should either produce 3' protruding ends or blunt ends at the broken junctions of the DSB. Alternatively, the DSB event, especially resulting from use of a nuclease, especially a meganuclease, could further involve the use of additional means, in particular of a compound such as an enzyme, which enables 5' protruding ends to be modified into 3' protruding ends or into blunt ends.

I-Scel is an example of an endonuclease which generates 3' protruding ends.

In a case where the DSB event, especially resulting from the use of a nuclease, in particular a meganuclease, provides originally 5' protruding ends, a further enzyme (either a cellular enzyme or an added enzyme) may be involved, which enables digestion of 5' protruding nucleotides in order to achieve the preparation of 3'protruding ends or blunt ends. Such an enzyme may be a 5'exonuclease, or a 3'polymerase, or a combination of an helicase and an endonuclease. These enzymes can be endogenous or brought into the cell by conventional molecular biology methods (i.e. Co-transfection of TdT expressing plasmid with a plasmid coding for a 5'exonuclease or a 3'polymerase). As examples, illustrating this embodiment, 5'exonuclease EXO1 can be cited or the combination of a helicase of the recQ family in association with the 3' polymerase Dna2. Examples of various rearrangements at the ends of DNA extremities have been disclosed in relation to translocation points in human cells (Zucman-Rossi et al, 1998).

In a particular embodiment of the invention, the broken ends resulting from DSB are obtained from a single event.

In another particular embodiment, the broken ends resulting from the DSB are obtained from multiple events especially from DSB at 2 locations in the polynucleotide.

It is therefore possible to perform double-strand break, especially targeted DSB, in one or more than one locus of one or more polynucleotide(s) of interest, said polynucleotide being either a targeted polynucleotide or a random polynucleotide as previously defined, in the chromosomal substrate of eukaryotic cells and accordingly to direct and generate junctional variability at one or multiple sites.

If control in the introduction of junctional variability in the eukaryotic cells is sought, when a nuclease, especially a meganuclease is used, the latter is expressed transiently or in a regulated manner in the cells after transfection or transduction of said cells with an expression vector comprising a transgene including the nuclease, especially the meganuclease, coding sequence or after transfection or transduction with the RNA transcript of the nuclease, especially the meganuclease gene or alternatively the nuclease, especially the meganuclease is delivered to the cell as a functional protein.

If control in the introduction of junctional variability in the eukaryotic cells is sought, TdT is expressed transiently or in a regulated manner in the cells especially after transfection or transduction of said cells with an expression vector (such as a plasmid) comprising a transgene including the TdT coding sequence or after transfection or transduction with the RNA transcript of a TdT gene or alternatively TdT is delivered to the cell as a functional protein.

A TdT coding sequence has been disclosed in the prior art and is especially found in the pMTdT plasmid of the patent application WO93/12228, deposited on December 10, 1991 under number CNCM I-1160 at the Collection Nationale des Cultures de Microorganismes (Paris France). The expressed TdT can be human TdT provided it is able to add N nucleotides to the 3'end of a nucleotide sequence, but it may also be from an animal such as a mouse.

Expression vectors for TdT and for meganuclease I-Scel have been disclosed in the prior art and especially include plasmid pCMV-TdT or pCMV-I-Scel as disclosed by (Liang et al, 1998).

When TdT or the nuclease, especially the meganuclease, is brought into the cell as a functional protein, its activity in the cell is fully controlled as it remains transient.

As previously defined, in order to be functional, the nuclease, especially the meganuclease, must be able to recognize a recognition sequence in a polynucleotide in the chromosomal context. If such a recognition sequence is not naturally present in said polynucleotide, the nuclease, especially the meganuclease, recognition site must be engineered or inserted in the polynucleotide of interest especially in the target polynucleotide. In such a case engineering or insertion of the recognition site may be performed on the polynucleotide in the chromosomal context or before said polynucleotide is inserted in the cell, or prior to its integration into the chromosomal environment of the cell or after said introduction and integration.

The nuclease, especially the meganuclease, must be able to cleave the DNA at a cleavage site in a polynucleotide in a chromosomal context. According to a particular embodiment, the cleavage site of the nuclease, especially the meganuclease, is naturally present in the sequence of the polynucleotide of interest. Alternatively, if such a cleavage site is not naturally present in said polynucleotide, a cleavage site must be engineered or inserted in the polynucleotide. In such a case engineering or insertion of the cleavage site may be performed on the polynucleotide of interest in the chromosomal context or before said polynucleotide is inserted in the cell, or prior to its integration into the chromosomal environment of the cell or after said introduction and integration. In such embodiments, the recognition site of the nuclease, especially the meganuclease can concomitantly be engineered or inserted in the polynucleotide.

In an embodiment of the invention, where the recognition site of the nuclease, especially meganuclease, is engineered or inserted in the polynucleotide, a further nuclease, especially meganuclease, may be added after DSB and before the action of TdT, said further nuclease, especially meganuclease, being selected for its capacity to digest the recognition site after DSB.

The eukaryotic cells which are used to carry out the method of the invention may be any type of eukaryotic cell, as disclosed herein. In a particular embodiment, said cells are especially cells which do not naturally express TdT and are especially non lymphoid cells, or more particularly are not pre-B or pre-T cells where TdT is naturally active in a time space well-known frame.

In a particular embodiment, the cells are mature cells or differentiated cells. In another embodiment, the cells are immature cells and especially are progenitor cells i.e. cells having a restricted level of specialisation toward a particular lineage and are capable of proliferation. The cells may be or include pluripotent cells. The cells can also be stem cells, including adult stem cells or embryonic stem cells to the extent that the latter are obtained without requiring embryo destruction, when said cells are human cells.

The eukaryotic cells which are subject to the method of the invention can be any eukaryotic cells which can be manipulated *ex vivo.* Among these cells, the invention especially relates to cultured cells, primary cells which are obtained from a tissue or from an organ, secondary cells which have undergone step(s) of cultivation, cell lines, and differentiated tissues.

In a particular embodiment, the cells selected to perform the method of the invention are wild-type cells. In another embodiment, the cells are modified cells, as a result of manipulation, including genetic manipulation, or as a result of contact with agents or organisms such as pathogenic organisms including viruses which modify their phenotype and/or their genotype. In a particular embodiment the cells are especially recombinant cells.

In a particular embodiment, the cells selected to perform the method of the invention are mutated and/or naturally deficient or rendered deficient in at least a further particular nucleic acid of interest, especially a gene, distinct from a gene coding for TdT, or for the Ku80 protein or the XRCC4 protein. This particular embodiment allows the production of at least double-mutants through the method of the invention when the polynucleotide of interest has been mutated as a result of performance of said method.

The cells used to perform the invention may be of the same type or may be a collection of heterogeneous cells, i.e., a collection wherein all the cells do not have the same phenotype.

In a preferred embodiment, the eukaryotic cells are yeast, fungus, plant cells, or are fish cells or birds cells.

In a preferred embodiment, the eukaryotic cells are from Vertebrates, especially mammalian cells, in particular human cells, murine cells, especially mouse or rat cells.

For illustration purposes, the following cells are cited for the purpose of performing the invention:

- myeloid cells, especially human myeloid cells that may be manipulated ex vivo according to the invention, prior to administration to a host;

- murine carcinoembryonic cells for the development of animal models.

In a particular embodiment of the invention, the polynucleotide of interest is in the chromosomal context in the cell, and is contained in a sequence of a gene, either in a coding or in a non coding sequence, especially in regulatory sequence such as a promoter, or is contained in a post translational active sequence. As earlier stated, the polynuclotide of interest may be heterologous to the chromosomal substrate of the cell or may originate from said chromosomal substrate, possibly after modification of the native sequence.

As examples of polynucleotides of interest, the invention provides nucleic acids consisting in or contained in:
- a gene expressing an enzyme, such as a kinase, in particular wherein the sequence of the polynucleotide of interest encodes the active site of the enzyme,
- a gene expressing a cell receptor,
- a gene expressing a structural protein, a secreted protein, such as a cytokine, or a regulatory protein, including for example an interleukin or interferon,
- a polynucleotide, especially a gene of a pathogen such as a virus a bacterium or a parasite,
- regulatory sequences for transcription or for expression of said genes.

The invention thus also relates to a method of creating junctional variability in the nucleotide sequence of a polynucleotide of interest thereby providing mutated polynucleotide comprising:
a) implementing the method of the invention on a polynucleotide of interest,
b) recovering cells comprising the polynucleotide of interest which has been recombined as a result of said method and, optionally,
c) recovering said mutated polynucleotides.

In a particular embodiment the step c) hereabove is replaced by, or is followed by, a step of recovery of the expression products of the mutated polynucleotides.

The invention also concerns a library of eukaryotic cells, which is obtained by implementing the method as disclosed in the present application and performed on a population of eukaryotic cells.

The invention also relates to a collection of recombinant clones obtained by performing the steps of:
a) performing the method of the invention on a population of eukaryotic cells,
b) recovering recombinant clones from said cells wherein said each clone comprises the polynucleotide of interest having undergone mutagenic repair.

The method of the invention also allows the determination of occurrence(s) of generation of DSB(s) in a cell or in a population of cells. Thus, the invention also relates to a method for determining occurrence(s) of generation of double-strand breaks(s) in a cell, comprising the steps of:
a) performing the method of generating junctional variability in the nucleotide sequence of a polynucleotide of interest of the invention, according to any one of its embodiments as disclosed herein,
b) evaluating the junctional variability generated in said cell.

By "determining the occurrence of generation of DSB(s)" it is understood the assessment of junctional variability obtained after occurrence of a DSB generated by the method of the invention. Such assessment extends to measurement of the amount of DSBs generated by the method of generating junctional variability of the invention.

The method for determining occurrence(s) of generation of double-strand breaks(s) in a cell thus encompasses both a qualitative and a quantitative determination of the presence of DSB event(s) in a cell. According to a particular embodiment, the method for determining occurrence(s) of generation of double-strand breaks(s) can be used for the purpose of assessing the efficacy or efficiency of the generation of double-strand breaks(s) in a cell.

In a particular embodiment, DSB(s) can be generated by at least a nuclease, especially a meganuclease, and the efficacy or efficiency of the latter is evaluated.

Consequently, the method for determining occurrence(s) of generation of double-strand breaks(s) can be used for the purpose of quantifying the efficiency of such a nuclease, especially meganuclease, whose recognition site was introduced, engineered, or naturally present in the genome of the cell. As detailed hereafter, TdT does not interfere with the C-NHEJ pathway (the end-joining efficiency is not affected by the presence of TdT) and N-nt additions mainly appear to be very efficient at DSBs locus (70% of efficiency in wild-type KA8 cells). In addition the present invention is of interest to reveal high-fidelity repairs of DNA at the level of DSB(s).

According to a preferred embodiment, the evaluation of the generated junctional variability of step b) can involve a step of amplification of the DNA of the cell resulting from step a), e.g., by Polymerase Chain Reaction (PCR) or equivalent methods. More specifically, such amplification might be directed to a DNA sequence including the sequence around the generated DSB(s). The amplified DNA can be the genome of the cell, and/or one or several regions in said genome, especially targeted regions, and/or in particular regions containing the recognition site of one or several nuclease(s), especially meganuclase(s), and/or random regions having a statistical value. The amplification should target at least one sequence harbouring junctional variability. In a particular embodiment, the amplification can be directed to a DSB cleavage site.

The evaluation of the generated junctional variability may further involve the characterisation of the result of such amplification, i.e. in order to determine if modifications have occurred in the DNA sequence, especially if a DSB locus was modified in comparison with the locus in the original DNA sequence. Such characterisation can be performed through known molecular biology methods, such as, but not limited to, Southern Blotting, cartography involving restriction enzymes or direct sequencing of the DNA.

The evaluation of the generated junctional variability may encompass qualitative or quantitative comparison, especially by any molecular biology method, between the DNA which was subjected to the method of the invention and the DNA of a control. Alternatively, the evaluation of the generated junctional variability may involve direct qualitative or quantitative analysis of the DNA which was subjected to the method of the invention, for example by sequencing or deep sequencing. Sequencing or deep sequencing may also be performed on PCR products, partly digested or not by a nuclease, especially a meganuclease, if such an enzyme was used.

The invention further concerns the use of Terminal deoxynucleotidyl transferase (TdT) as a marker of DSB events, wherein a DSB is repaired in a way generating junctional variability at the locus of said DSB.

The use of TdT as a marker is made in accordance with any one of the embodiments contained in the present description. In a preferred embodiment, TdT is used as marker in a eukaryotic cell which is competent for canonical Non Homologous End Joining pathway (NHEJ) repair and which has been used to perform the method of the invention. Such cells can be non-lymphoid cells. In a particular embodiment, DSB(s) can be generated by nuclease(s), especially meganuclease(s). DSB(s) can be generated on targeted regions of the genome.

As a specific exemple of the use of TdT according to the invention, competent cells can be co-transfected with vectors containing sequences for a nuclease, especially a meganuclease, and for TdT. A PCR is then performed to amplify sequences around cleavage site of the nuclease, especially meganuclease. PCR products can be further sequenced or digested *in vitro* by the nuclease. In such a case, only non-digested PCR products are further sequenced.

Other features and preferred embodiments of the invention will appear in the examples and drawings which are illustrative of the way the invention can be performed.
**Figure 1****:** chromosomal substrate: pCMV-H2Kd-CD8-CD4 vector used for stable integration into the cell genome. The sequence comprises 2 recognition sites for I-Scel enzyme. Different strategies for repair events are depicted: in the first one only I-Scel is delivered to or expressed in the cells. In the second strategy both I-Scel and TdT are delivered to or expressed in the cells. The result of the broken junctions after cleavage by I-Scel and repair events are shown. Sequences corresponding to SEQ ID NO: 111 to SEQ ID NO: 113 are disclosed in this Figure.
**Figures 2A and 2B****:** expression of TdT and I-Scel in XCC4-deficient cells. (A): Western-blot and (B) immunofluorescence analysis. Only XRCC4-deficient cells are shown. However, similar results were obtained for all the other cell lines used in this study.
**Figures 3A** **and** **3B****:** sequence analysis of the junctions in wild-type (A) and Ku-deficient (B) cells. Clones SEQ ID NO: 1 to SEQ ID NO: 54 are disclosed in these Figures.
**Figures 4A** **and** **4B****:** sequence analysis of the junctions in XRCC4-deficient cells (A) and XRCC4-complemented cells (B). Clones SEQ ID NO: 55 to SEQ ID NO: 109 are disclosed in these Figures.
**Figure 5****:** model of N-nucleotides addition by TdT in DNA repair-proficient cells at non V(D)J DSB via the C-NHEJ.

### Examples

In order to assess the mutagenic potential of TdT in a non-V(D)J DSBs, determine its potential for applications to generation of variability in nucleic acids and understand the discrepancies between the results obtained with animal models and plasmid substrates, the inventors used a chromosomal substrate that allows the study of the junctional variability introduced by TdT in Ku80 or XRCC4-deficient cells and their respective control.

The obtained results show that, in a chromosomal context, N nucleotides (non-templated nucleotides) are efficiently added to non V(D)J DSBs and that this process is Ku and XRCC4-dependent, suggesting that a simple deregulation of TdT expression would be sufficient to be highly mutagenic at the DSB.

### Materiel and Methods

### Cells and transfection

Chinese hamster ovary (CHO) XR-1 radiosensitive mutant cell lines (Bryans et al., 1999) and their derivatives were cultured in DMEM (- pyruvate), and CHO-K1, xrs6, and their derivatives were cultured in α-MEM, supplemented with 10% FCS, 2mM glutamine, and 200 international units/ml penicillin at 37°C with 5% CO₂. Cells (2x10⁵ for xrs6 and 3x10⁵ for XR-1) were plated one day before the transfection with Jet-PEI, under the conditions specified by the manufacturer (Q-BIOgene). Ku-deficient cells and the corresponding control cell lines have been transfected with (1) 10x10⁻¹³ moles of pBEL, an empty vector (mock experiment); (2) 2.5x10⁻¹³ moles of pBEL and 7.5x10⁻¹³ moles of pCMV-I-Scel (Liang et al., 1998) (Rouet et al., 1994), the expression vector of the meganuclease I-Scel; (3) 7.5x10⁻¹³ moles of pCMV-I-Scel and 2.5x10⁻¹³ moles of pCMV-TdT, the expression vector of TdT (Boulé et al., 1998) (Doyen et al., 2003) (pMTdT plasmid of the patent application WO93/12228, deposited on December 10, 1991 under number CNCM I-1160 at the Collection Nationale des Cultures de Microorganismes (Paris France)). XRCC4-deficient cells have been transfected with (1) 10x10⁻¹³ moles of pBEL; (2) 2.5x10⁻¹³ moles of pBEL and 7.5x10⁻¹³ moles of pCMV-I-Scel; (3) 1.25x10⁻¹³ moles of pBEL, 7.5x10⁻¹³ moles of pCMV-I-Scel and 1.25x10⁻¹³ moles of pCMV-TdT; (4) 1.25x10⁻¹³ moles of pBEL, 7.5x10⁻¹³ moles of pCMV-I-Scel and 1.25x10⁻¹³ moles of XRCC4 cDNA for complementation experiment; (5) 7.5x10⁻¹³ moles of pCMV-I-Scel and 1.25x10⁻¹³ moles of TdT and 1.25x10⁻¹³ moles of XRCC4. The pBEL plasmid was constructed from a pcDNA3 plasmid, according to the construction described in Lambert et al., 2000.

### Western blotting

The level of TdT and I-Scel expression in the different cell lines for each transfection condition was assessed by Western blot. Forty-eight hours after transfection, cells were detached with PBS/EDTA 0.5 mM, washed with PBS and lysed in Laemmli buffer. Samples (30 µg of total cellular protein) were electrophoresed through 10% SDS-polyacrylamide gels, transferred to PVDF, blocked for 1 hour in 5% milk/TBS-T. Blots were probed overnight at 4° C for TdT with a polyclonal rabbit antibody (Abcam, ab14772) diluted 1/1000 in 5% milk/TBS-T followed by an incubation with peroxydase (HRP)-conjugated anti-rabbit IgG, and for I-Scel with an anti-HA antibody (Covance) diluted 1/500 followed by an incubation with HRP-conjugated anti-mouse IgG. Equal loading was determined using monoclonal anti-actin antibody for 1 hour followed by a 45 min incubation with HRP-conjugated anti-rabbit IgG. Antibodies were detected using enhanced chemifluorescence (ECF) (Amersham).

### Immunofluorescence

Forty-eight hours after transfection, cells on coverslips were washed in PBS and fixed with PBS/2% PAF for 15 min at room temperature. After 10 min of permeabilization with PBS/0.5% saponin and a saturation step with PBS/0.5% saponin/ 0.2% BSA for 30 min, cells were incubated for 1 hour with polyclonal rabbit TdT antibody (Abcam, ab14772) and monoclonal mouse anti-HA antibody (Covance) for the detection of I-Scel. Both antibodies were diluted 1/250 in PBS/0.5% saponin/ 0.2% BSA. Cells were washed with PBS/0.1% saponin and then incubated for 1 hour with the secondary antibodies diluted 1/400 in PBS/0.5% saponin/ 0.2% BSA (anti-S3 for I-Scel and anti-S4 for TdT). After a washing step with PBS/0.1% saponin and cells were stained with DAPI (1/1000) before the mounting of the coverslips.

### FACs analysis, Microscope analysis, Enrichment of CD4⁺ expressing cells, Junction sequence analysis, Statistical analysis

All these manipulations were performed as previously described in ref (Guirouilh-Barbat et al., 2004) (Guirouilh-Barbat et al., 2007).

### Results

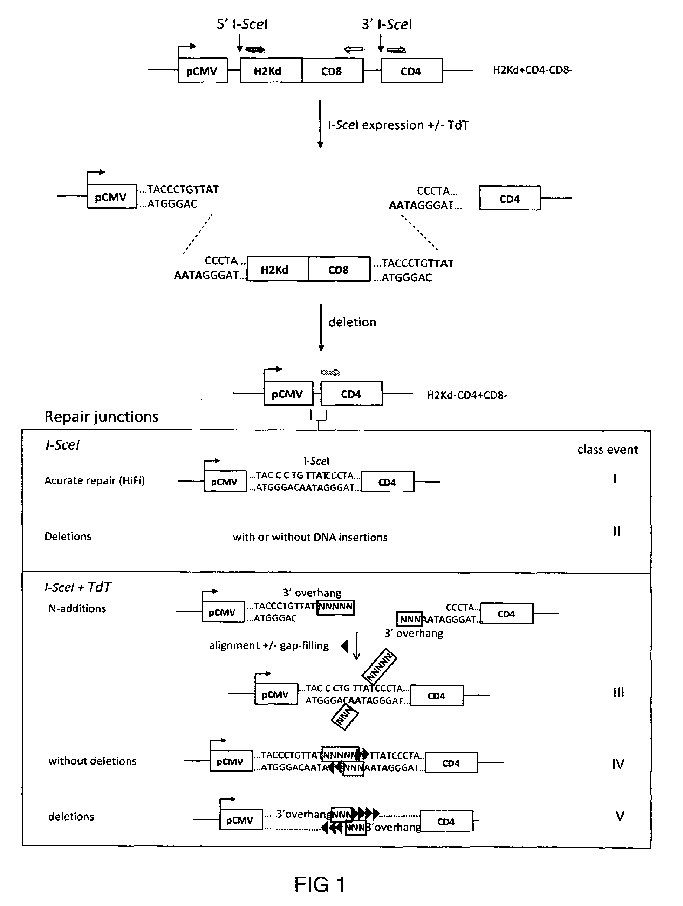

### 1. Cell lines and strategy (Fig 1)

To analyse the junctional variability associated to TdT in a non V(D)J but chromosomal context, we used the chromosomal substrate depicted in Fig 1, stably integrated as a single copy, into the genome of CHO-K1 (wild-type), xrs6 (Ku-defective) or XR-1 (XRCC4-defective) hamster cells. The digestion of both I-Scel sites generates 3' overhangs to which TdT can add N-nucleotides. The resulting excision of the H2KD-CD8 fragment leads to CD4 expression that is monitored by FACS analysis and repair junctions are analyzed by sequencing. With fully complementary extremities generated by I-Scel, we expected to observe in wild-type cells, in the absence of TdT, a high frequency of accurate repair events (Fig1, class I) and few junctions with nt deletions and possibly DNA capture (Fig1, class II) as we have previously shown (Capp et al., 2006) (Capp et al., 2007) (Guirouilh-Barbat et al., 2004) (Guirouilh-Barbat et al., 2007). In contrast, in the presence of TdT, if the addition of N nucleotides to one or both 3' overhangs is efficient, we would foresee that faithful repair should be less frequent, except if the extremities are often repaired by annealing of the 4 protruding nucleotides (4 P-nt) indicated as TTAT in Fig1, resulting then to the loss of the newly added nucleotides (Fig1, class III).

The complementarity between N nt added to each DNA extremity that was exposed to exonuclease activity (nt deletions) or not, leads to an alignment of the extremities followed by a gap-filling process mediated by a polymerase (nucleotides indicated as « NNNNN » in Fig1) before the final ligation step (Fig1, class IV and V).

The different cell lines used to characterize the mechanism by which TdT adds N nucleotides are as follows.

| Parental cell line | Cell lineage | Deficient protein |
|---|---|---|
| CHO-K1 | KA8 | - (control) |
| Xrs6 | XD11 | Ku86 |
| XR-1 | XCO11 | XRCC4 |

### 2. TdT does not interfere with the repair efficiency in wild-type, Ku or XRCC4-deficient cells

To induce DSBs and promote N-addition at the breakpoint junctions, cells were co-transfected with expression vectors for I-Scel and for TdT. For the control experiment, without TdT, cells were co-transfected with the I-Scel expression vector and an empty vector.

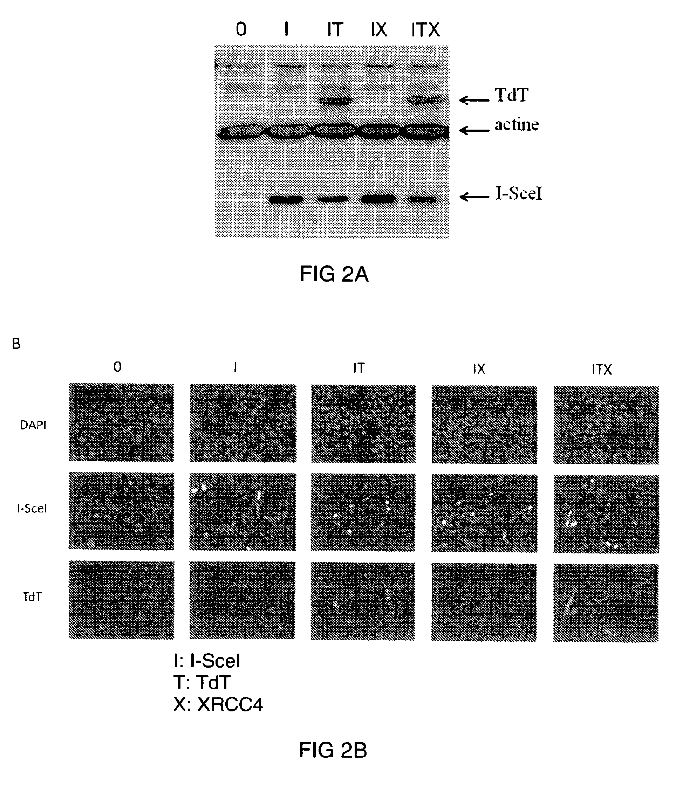

Then, we first tested different mole ratio for I-Scel and TdT vectors. We monitored the levels of I-Scel and TdT proteins by Western blot for the different transfection conditions, in presence or absence of TdT. As shown in Fig 2A for XRCC4-deficient cells"I-Scel expression when the vector is co-transfected with TdT (IT) is at least 2 fold weaker than when I-Scel is co-transfected with the empty vector (I). Likewise, when cells are transfected with XRCC4 gene for complementation, I-Scel is less expressed in the presence of TdT vector, which suggests a competition between both plasmids. Although not shown, data are similar in all other cell lines.

Then, we checked the co-transfection efficiency at the cellular level. Immunofluorescence staining showed that for the transfection condition we used, most cells expressing I-Scel were also TdT positive (Fig2B, conditions IT and ITX).

We investigated the effect of TdT on the efficiency of distal end-joining by measuring the rate of CD4+ cells in the different cell lines in presence or absence of TdT. The frequencies of CD4+ cells we found for the control experiments, without TdT, are remarkably close to those reported in our previous studies (Guirouilh-Barbat et al., 2004) (Guirouilh-Barbat et al., 2007). However, as previously shown in Fig 2A, since the level of I-Scel decreased almost by half in the presence of TdT, our results rather suggest that TdT has no influence on the repair efficiency in all the different genetic backgrounds. The very high efficiency of co-transfection with I-*Sce*l and TdT expression vectors allows a strong investigation of the potential effect of TdT on junctional variability.

### 3. TdT efficiently adds N nucleotides to chromosomal DSBs only in wild-tyye and XRCC4-complemented cells.

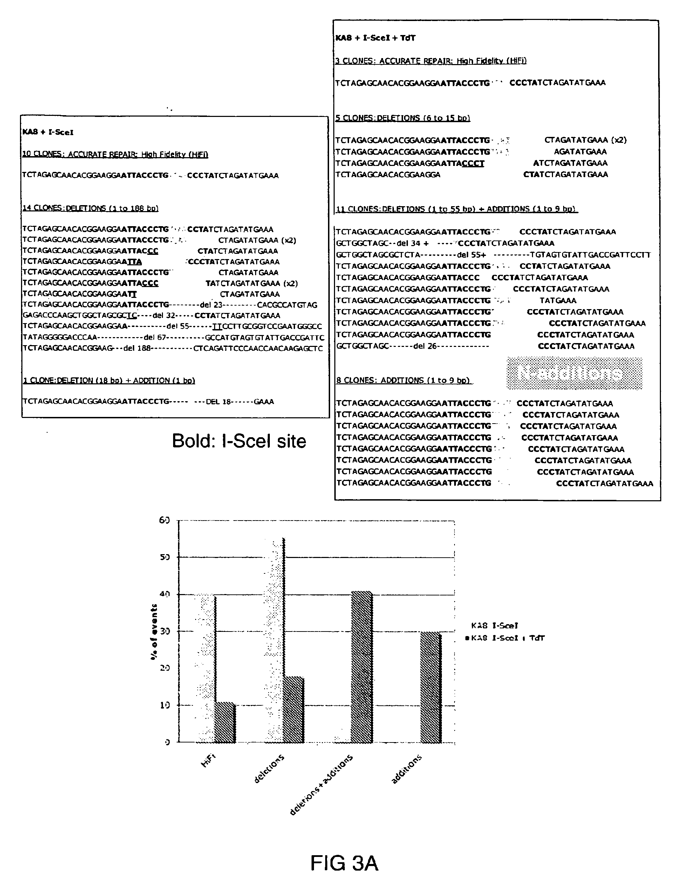

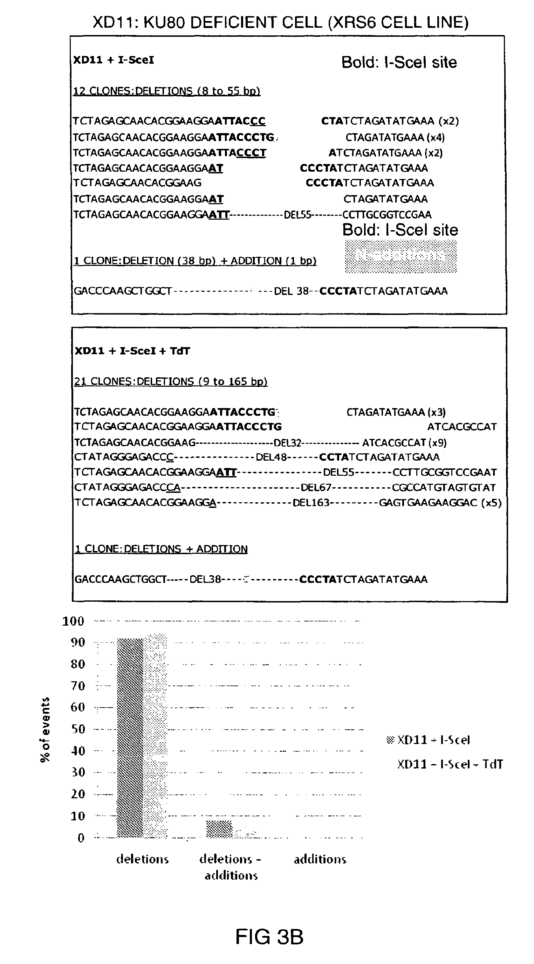

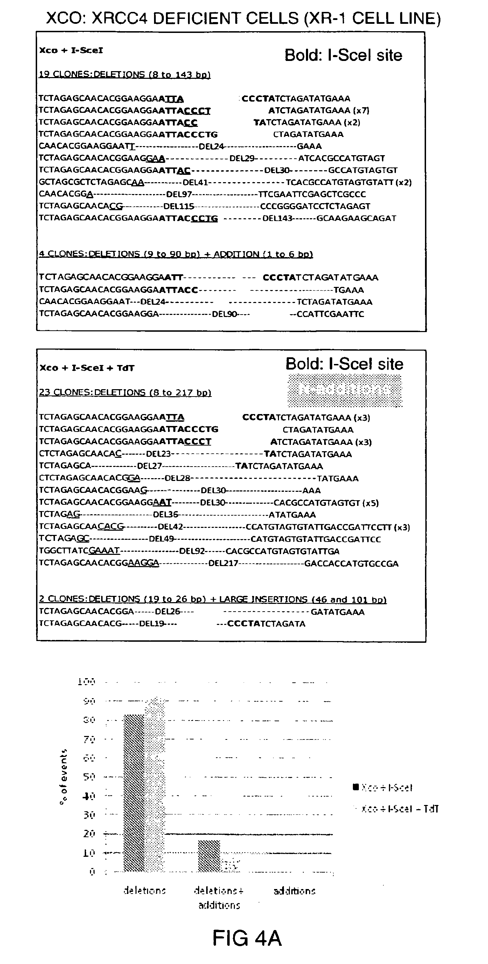

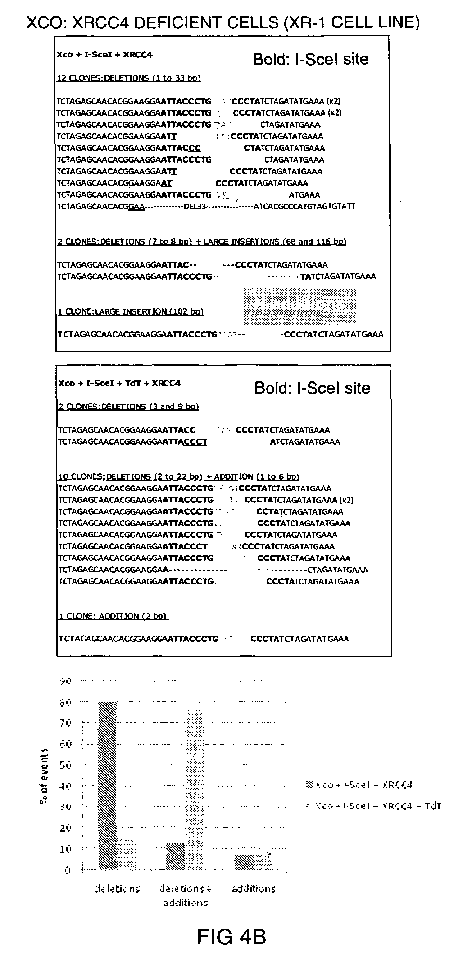

To determine whether N-addition by TdT is Ku and/or XRCC4-dependent we transfected the canonical NHEJ-deficient cell lines and their respective controls with I-Scel, in the presence or absence of TdT. Repair junctions are shown in Figures 3A and 3B and 4A and 4B.

In agreement with our previous studies (Guirouilh-Barbat et al., 2004) (Guirouilh-Barbat et al., 2007), we found that in the presence of I-Scel the frequency of junctions with nt deletions is much higher in Ku80-deficient cells compared to wild-type cells, respectively 100 and 60% of the repair events (Fig.3B upper panel and Fig 3A left upper panel). We did not observe any accurate repair in Ku80-deficient cells among the 13 repair events sequenced (Fig 3B, upper panel) whereas in wild-type cells, 10 junctions out of 25 (40%) were error-free (Fig 3A, left upper panel). Likewise, junctions in XRCC4-deficient cells present more nt deletions than in complemented cells (Fig.4A and 4B)

Most importantly, in the absence of TdT, additions of nucleotides at the junctions are rare events for all cell lines, i.e. whatever the repair capacity of cells. We found 1 nt insertion for 1 clone out of 13 (8%) in Ku-deficient cells and 1 clone out of 25 (4%) in wild-type cells (Fig 3B upper panels and Fig 3A left upper panel). In XRCC4-deficient background, 4 repair events out of 23 (17%) presented nucleotides addition ranging from 1 to 6 bp (Fig 4A, upper panel). When these cells are complemented, 20 % (3 repair events out of 15, Fig. 4B, upper panel) of the junctions exhibit insertions of 68, 93 and 116 bp that are more likely DNA capture events. Several TdT-independent mechanisms have been proposed to account for these extra-nt additions (Roth et al., 1985) (Roth et al., 1989) (Roth et al., 1991).

In contrast, addition of TdT dramatically increased the number of junctions with N additions in wild-type and XRCC4 complemented cells: from 4 to 70 % and from 0 to 85 %, respectively (Fig 3A and 4B).

Surprisingly, in contrast with the results of a study based on a plasmid assay (Sandor et al., 2004), we did not find any N-addition in Ku-deficient cells. Only 1 clone out of 22 (5%) presented an addition of 1 bp compared to 19 clones out of 27 (70%) for the corresponding control cells (Figs 3B and 3A). Likewise, we did not find any N-addition in XRCC4-deficient cells. Only 2 clones out of 25 (8%) presented insertions of 46 and 101 nt, like the complemented cells in the absence of TdT (Figs 4A and 4B).

Thus, the addition of TdT has no effect on the frequency of the different repair patterns (HiFi, deletions, deletions + N-additions) in Ku and XRCC4-deficient cells, whereas in repair proficient cells, addition of N nt decreases the frequency of error-free events: from 40 to 11 % in wild-type cells (Fig 3A) and from 50 to 35% in XRCC4 complemented cells (Fig.4B).

Our data clearly show that, in a chromosomal non-V(D)J DSB, TdT promotes N-addition and TdT acts in a KU- and XRCC4-dependant manner.

Interestingly, in wild-type cells (Fig 3 A), the N-additions conserve all or part of the four 3'-protruding nucleotide of the site cleaved by I-Scel in a majority of cases. Noteworthy, the conservation of the I-Scel site (even partial) is a hallmark of the KU/XRCC4 pathway (Guirouilh-Barbat et al., 2007). Limited deletions extent is also a hallmark of the KU NHEJ pathway (Guirouilh-Barbat et al., 2004) (Guirouilh-Barbat et al., 2007). In absence of KU80, while the frequency of end-joigning remains very high compared to wild-type cells, the frequency of accurate events is totally abolished and the use of the four 3'protruding nucleotides disappeared in all events but one (Fig 3 B).

### Discussion

TdT, whose physiological role is to increase the diversity of the immune repertoire, by adding non-templated nt to V(D)J junctions, is "mutagenic" *per se.* Its expression is restricted to B and T cells at a particular stage of their development.

### TdT and end-joining efficiency

First, TdT barely affects the efficiency of the joining process. Indeed, for all cell lines tested, whether they are repair-proficient or repair-deficient, the frequency of CD4+ cells, ie., the joining efficiency, is reduced by only 2 times when TdT is added, even for the cell lines carrying the substrate with the I-Scel sites in opposite orientation (data not shown). This is very likely due to a weaker expression of I-*Sc*el gene when co-transfected with TdT vector. Our result fits very well with the one of Sandor *et al* (Sandor et al., 2004) who have detected with a chromosomal substrate similar to the one we used around 2 times less joining events when TdT is added. In addition, the fact that the frequency of lg and TCR V(D)J gene rearrangement is not affected in TdT deficient mice (Komori et al., 1993) or in mice expressing TdT constitutively (Bentolila et al., 1997) strongly supports that TdT has no effect on the repair efficiency but rather only on adding junctional variability.

### TdT and wild-type context.

Importantly, in wild-type cells, we found a frequency of N addition by TdT in non-V(D)J junctions at level similar to that of V(D)J junctions (Shimizu and Yamagishi, 1992) (Iwasato and Yamagishi, 1992). Thus, we show that TdT, in a chromosomal context, can efficiently add extra-nt to random DSB and that this process does not require any lymphoid specific factor. In addition, as previously shown (Gerstein and Lieber, 1993) (Lewis, 1994b) (Repasky et al., 2004), we also observed a decrease of the extent of deletion in presence of TdT, suggesting that TdT impairs the efficiency of A-NHEJ. Moreover, if we only take into account the junctions without N-additions then microhomology usage is the same with or without TdT, which also suggests that the A-NHEJ is not increased when TdT is expressed.

Likewise, we also observed in XRCC4-complemented cells a decrease of nt deletion at DNA ends when TdT is added. The repair efficiency being almost the same in presence or absence of TdT, it is unlikely due to a delay in the repair of DNA. One explanation is that TdT binds to DNA ends and that its association with Ku80 may be more efficient to prevent the access to exonucleases than can do Ku proteins alone. Indeed, it has been proposed that TdT stabilizes the canonical NHEJ machinery by binding to DNA ends *via* its interactions with the different partners (Mahajan et al., 1999).

### TdT and Ku-deficiency

Importantly, we did not detect any N-addition by TdT in Ku-deficient cells. Although this is consistent with mice models, our result contrasts sharply with Sandor's study that has been done in the same cell line and where they frequently observed abnormally long nt insertions in presence of TdT (Sandor et al., 2004). However, it is noteworthy that they used an episomic plasmid substrate that already showed in the same Ku and XRCC4-deficient cell lines few results that contrast with the ones we obtained with the chromosomal substrate (Kabotyanski et al., 1998) (Guirouilh-Barbat et al., 2004) (Guirouilh-Barbat et al., 2007). For example, precise rejoining of DNA ends is not impaired in Ku80-deficient cells with a plasmid substrate (Kabotyanski et al., 1998) whereas it is highly affected at a chromosomal level (Guirouilh-Barbat et al., 2004) (Guirouilh-Barbat et al., 2007). Likewise, it has also been shown that the average length of N nt added by TdT is smaller with a chromosomal substrate compared to an episomal substrate (Repasky et al., 2004). Reasons that can explain such differences include the following. First, the DNA structure of extra-chromosomal substrates is probably different from the chromatin. Second, it is formally possible that some recombination of plasmids occurs outside the nucleus, i.e. in the cytoplasm. However, our data provide conclusive evidence that TdT even when overexpressed and efficiently targeted to the nucleus (immunofluorescence staining, Fig 2) cannot add N nt in the absence of Ku proteins, which is consistent with mice models.

Purugganan et al. (Purugganan et al., 2001) who also did not observe in Ku-deficient cells any N addition in recombinant junctions using a V(D)J episomal substrate proposed 3 models to explain the absence of N regions. First, they proposed that N nt could be added but that the resulting 3' overhangs could not be joined in the absence of Ku80. Alternatively, TdT would add N nt aberrantly in the absence of Ku80 forming intermediates containing long 3' extensions that cannot be joined efficiently. This model is attractive in the way that it could explain the discrepancy between plasmid and chromosomal substrates: the long intermediates would interfere with the repair machinery at a chromosomal level, but not with plasmids. However, in that case, the joining efficiency would have dramatically decreased when TdT is added, which is not what we observed. That is why, in accordance with Purugganan et al. who have also shown P nt insertions in Ku-deficient cells (Han et al., 1997) (Bogue et al., 1997; Purugganan et al., 2001) and a lack of difference in the length of products generated by TdT in Ku80-deficient and wild-type cell extracts (Purugganan et al., 2001), we also argue against this model.

Second, it has been proposed that TdT could add N nt in the absence of Ku80 but that these would be then removed by excessive nuclease activity. Although the fact that a large number of coding joints which lack N regions from Ku80^{-/-} mice and hybrid joints formed in Ku-deficient cells were not deleted from either end, our observation of a dramatic increase of nt deletion size at the junctions (see below) do not exclude this model. Alternatively, the extra-nt would disappear as a consequence of the joining process via microhomologies flanking the N region. However, the decrease of microhomology usage that we observed at the junctions from Ku-deficient cells does not support this scenario. Furthermore, even in wild-type cells where N addition is very efficient and where the deletions were limited, offering thus the possibility of annealing between the 4 P-nt from both I-Scel sites (Fig1A, case III), only 11 % of the repair events (Fig3A, 3/27 clones) could account for such a model, which is not very relevant. However, this is probably due to the fact that canonical NHEJ does not make use of microhomogy. In addition, again, the presence of P nt at junctions from Ku-deficient cells argues against this model.

The third scenario proposed by Purugganan et al. (Purugganan et al., 2001) is the recruitment model: Ku would recrute TdT to the repair complex. In its absence, TdT could not be recruited, blocking the addition of N additions. However, several observations argue against this model. First, *in vitro* studies have clearly shown that TdT can bind to oligonucleotides in the absence of Ku. In addition, the decrease of microhomology usage in Ku-deficient cells when TdT is added (see below) strongly suggests that the polymerase has access to the repair machinery complex even in the absence of Ku.

As we have previously shown (Guirouilh-Barbat et al., 2004) (Guirouilh-Barbat et al., 2007), deletions at DNA ends are more important in Ku-deficient cells than in control cells. However, we did not expect that TdT addition would increase the size of nt deletions. Two possibilities: either TdT cannot bind to DNA in the absence of Ku80 and thus protect DNA ends from exonuclease activity or, TdT binds to DNA but cannot prevent DNA degradation. However, the fact that in Ku-deficient cells TdT dramatically decreases microhomology usage, influencing thus the joining process, and the biochemical studies that show a binding of TdT to DNA even in absence of Ku proteins are in favor of the second scenario. However, how can we explain the decrease of microhomology usage in absence of N-addition? One possibility is that TdT interfere with the alternative NHEJ pathway. We have previously shown with the same chromosomal substrate that in the absence of Ku there is an alternative NHEJ process that uses microhomology at DNA ends. Thus, junctions generated in the absence of Ku80 but in the presence of TdT may be formed by a second alternative NHEJ pathway that does not make use of microhomology.

### TdT and XRCC4 deficiency.

If the implication of Ku proteins in N addition by TdT has clearly been suggested by the absence of N nt in the V(D)J junctions of Ku-deficient mice, it was more difficult to speculate about a potential role of XRCC4 as the deficiency for this protein is embryonic lethal (Gao et al., 2000). Here we have shown that N-addition by TdT is also XRCC4 dependent, which more strongly argues against the recruitment model of TdT by Ku (see previous part).

Moreover, as for Ku deficient cells, in presence of TdT, nucleotide deletion at chromosomal ends is also increased in XRCC4 deficient cells. One explanation would be that in the absence of XRCC4, the association of the complex Ku-TdT to DNA is unstable and then both proteins dissociate from the molecule that is then exposed to exonucleases before an alternative NHEJ machinery takes care of the repair. Indeed, a biochemical study has shown that only the complex Ku-XRCC4/ligase IV-TdT is stable (Mahajan et al., 2002). Thus, our analysis of the nucleotide deletion size at the junctions in the different genetic background suggests that indeed the repair complex is unstable when one of the partner, Ku or XRCC4, is missing.

However, in contrast with Ku deficient cells, we did not observe in XRCC4 deficient cells any influence of TdT on microhomology usage. Conversely, when the cells are complemented, like in wild-type cells, nt deletion at the extremities is decreased in presence of TdT and microhomology usage is the same whatever TdT is expressed or not. This suggests that an alternative NHEJ pathway, different from the one activated in Ku-deficient cells, may take place in XRCC4 deficient cells in presence of TdT.

Moreover, the cell lines used in the present study show that TdT does not require any lymphoid specific factor, but is potentially active in different tissues. These data show thus that TdT action is not restricted to V(D)J recombination, but more generally to enzyme-generated DSBs.
The requirement in KU80 and XRCC4 is consistent with the fact that the 3'protruding nucleotides generated by I-SCEI cleavage are, at least in part, maintained in most of the events exhibiting N-additions. Indeed, the use of the 3'-protruding nucleotide is a hallmark of the canonical KU/XRCC4-dependent NHEJ pathway (Guirouilh-Barbat et al., 2004) (Guirouilh-Barbat et al., 2007) (Rass et al., 2009). Since canonical KU80/XRCC4 NHEJ pathway is highly efficient even with mismatched ends (Guirouilh-Barbat et al., 2007) (Guirouilh-Barbat et al., 2004), TdT should facilitate annealing and re-sealing of the ends, by adding nucleotides. Nucleotides addition at both DNA ends prior ligation should result in duplication of the 3'protruding nucleotides interrupted by the N-additions, in absence of DNA degradation. Although such an event can occur, most of the N-additions events show the maintenance of 1 to 4 of the 3'-protruding nucleotides for at least one of the extremities strands and deletion on the other DNA end. This suggests that the two ends are processed separately prior to end-joining, and thus that the N-additions or the deletions occur prior the synapsis of the two ends. However, the requirement in XRCC4 for N-additions, strongly supports the idea that ligase 4 is necessary and thus, that the whole process from the early steps (KU80) to the late steps (XRCC4) acts according the canonical NHEJ pathway.

In absence of KU80 or XRCC4 no N-addition events were recorded. Several hypothesis can account for these results: 1- TdT is recruited at the DSB by the NHEJ machinery implying that both KU80 and XRCC4 are necessary for TdT recruitment. 2- N nucleotides could be added but the resulting 3' overhangs could not be joined in the absence of Ku80, as already proposed (Purugganan et al., 2001). Alternatively, TdT would add N nucleotides aberrantly in the absence of Ku80 forming intermediates containing long 3' extensions that cannot be joined efficiently. This model is attractive in the way that it could explain the discrepancy between plasmid and chromosomal substrates: the long intermediates would interfere with the repair machinery at a chromosomal level, but not with plasmids. However, in this case, the joining efficiency would have dramatically decreased when TdT is added, which is not what we observed. 3-TdT can add nucleotide in absence of KU80 and XRCC4 but nuclease activity removes the added nucleotides; this suggests that the nuclease activity should act from 3' to 5'. 4- TdT adds nucleotides but a 5' to 3' resection followed by annealing generate a Flap structure. The resolution of the Flap intermediate removes then the added nucleotides.

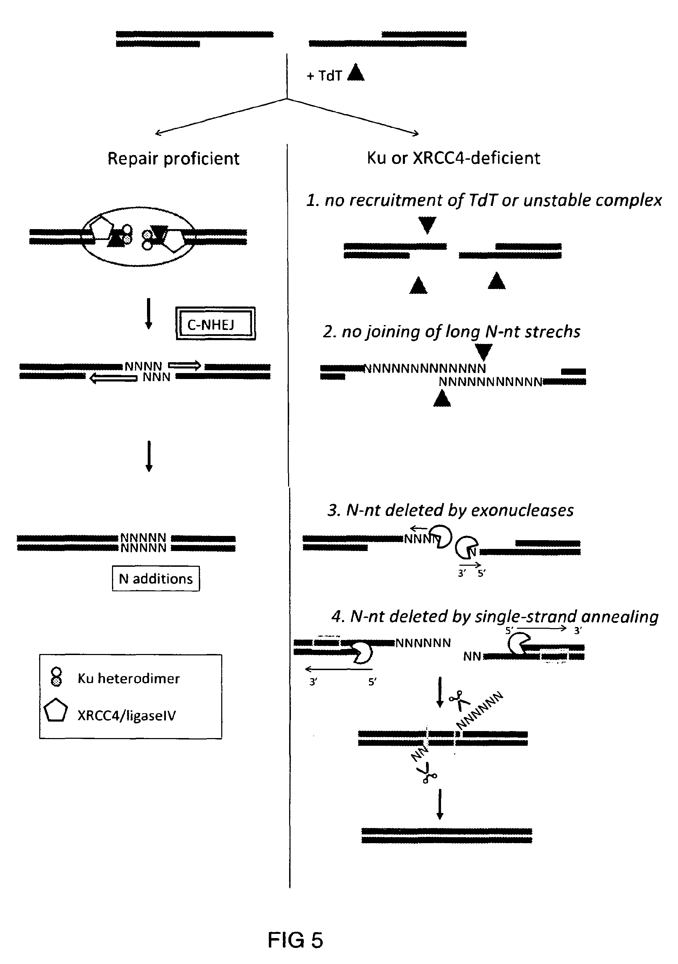

All together, our data show that TdT adds nt in a non-V(D)J chromosomal junction and that both Ku and XRCC4 are necessary for N addition by TdT, as summarized in Figure 5.

### References

1. Bangs, L.A. et al., (1991) J Immunol, 146, 1996-2004.
2. Benedict, C.L. et al., (2000) Immunol Rev, 175, 150-157.
3. Bentolila, L.A. et al., (1995) Embo J, 14, 4221-4229.
4. Bentolila, L.A. et al.,(1997) J Immunol, 158, 715-723.
5. Bogue, M.A. et al., (1997) Immunity, 7, 37-47.
6. Bollum, F.J. et al., (1978) Adv Enzymol Relat Areas Mol Biol, 47, 347-374.
7. Boule, J.B. et al., (1998) Mol Biotechnol, 10, 199-208.
8. Boule, J.B. et al., (2001) J Biol Chem, 276, 31388-31393.
9. Braziel, R.M. et al., (1983). Am J Clin Pathol, 80, 655-659.
10. Bryans M. et al., (1999) Mutat Res.;433, 53-58.
11. Buresh, C.J. et al., (2008) Am J Clin Pathol, 129, 894-898.
12. Capp, J.P. et al., (2006) Nucleic Acids Res, 34, 2998-3007.
13. Capp, J.P. et al., (2007) Nucleic Acids Res, 35, 3551-3560.
14. Chang, L.M. et al. (1986) CRC Crit Rev Biochem, 21, 27-52.
15. Cherrier, M. et al., (2008). Mol Immunol, 45, 1009-1017.
16. Chevalier, B.S. et al., (2001), Nucleic Acids Research, 29, 3757-3774
17. Corneo, B. et al., (2007) Rag mutations reveal robust alternative end joining. Nature, 449, 483-486.
18. Delarue, M. et al., (2002). Embo J, 21, 427-439.
19. Desiderio, S.V. et al., (1984). Nature, 311, 752-755.
20. Di Santo, et al., (2006), Curr Med Chem, 13, 2353-2368.
21. Doyen, N. et al., (2004). J Immunol, 172, 6764-6767.
22. Doyen, N. (1993) Nucleic Acids Res, 21, 1187-1191.
23. Feeney, A.J. (1990). J Exp Med, 172, 1377-1390.
24. Gao, Y. et al., (2000). Nature, 404, 897-900.
25.Gerstein, R.M. and Lieber, M.R. (1993). Nature, 363, 625-627.
26.Guirouilh-Barbat, J. et al., (2004). Mol Cell, 14, 611-623.
27. Guirouilh-Barbat, J. et al., (2007). Proc Natl Acad Sci U S A, 104, 20902-20907.
28. Han, J.O. et al., (1997) Mol Cell Biol, 17, 2226-2234.
29. Hasty, P. et al., (2008) Cell Cycle, 7, 1139-1145.
30. Iwasato, T. and Yamagishi, H. (1992), Eur J Immunol, 22, 101-106.
31.Jung, D. and Alt, F.W. (2004). Cell, 116, 299-311.
32. Kabotyanski, E.B. et al., (1998). Nucleic Acids Res, 26, 5333-5342.
33. Kato, K.I. et al., (1967). J Biol Chem, 242, 2780-2789.
34. Komori, T. et al., (1993). Science, 261, 1171-1175.
35. Lambert S. et al., (2000). The EMBO Journal, 19, 3090-3099.
36. Lewis, S.M. et al., (1994a). Semin Immunol, 6, 131-141.
37.Lewis, S.M. et al., (1994b). Adv Immunol, 56, 27-150.
38. Liang, F. et al., (1998). Proc Natl Acad Sci U S A, 95, 5172-5177.
39. Lieber, M.R. et al., (2004). DNA Repair (Amst), 3, 817-826.
40. Ma, Y. et al., (2004). Mol Cell, 16, 701-713.
41. Mahajan, K.N. et al., (1999). Proc Natl Acad Sci U S A, 96, 13926-13931.
42. Mahajan, K.N. et al., (2002). Mol Cell Biol, 22, 5194-5202.
43. Mathewson, R.C. et al., (1997). Pediatr Pathol Lab Med, 17, 835-844.
44. Murray, J.M. et al., (2006). J Immunol, 177, 5393-5404.
45. Nick McElhinny, S.A. and Ramsden, D.A. (2004). Immunol Rev, 200, 156-164.
46. Nourrit, F. et al., (1999). J Mol Biol, 292, 217-227.
47.Orazi, A., et al., (1994a). Mod Pathol, 7, 582-586.
48.Orazi, A. et al., (1994b). Am J Clin Pathol, 102, 640-645.
49. Paques, F. and Duchateau Ph. (2007). Current gene therapy, 7, 49-66*.*
50. Purugganan, M.M. et al., (2001). Nucleic Acids Res, 29, 1638-1646.
51. Rass E. et al., (2009) Nat. Struct. Mol. Biol., 16, 814-818.
52. Repasky, J.A. et al., (2004). J Immunol, 172, 5478-5488.
53. Robbins, D.J. et al., (1987) J Biol Chem, 262, 9494-9502.
54. Robbins, D.J. and Coleman, M.S. (1988). Nucleic Acids Res, 16, 2943-2957.
55. Rooney, S. et al., (2004). Immunol Rev, 200, 115-131.
56. Roth, D.B. et al., (1989). Mol Cell Biol, 9, 3049-3057.
57. Roth, D.B. et al., (1985). Mol Cell Biol, 5, 2599-2607.
58. Roth, D.B. et al., (1991). Nucleic Acids Res, 19, 7201-7205.
59. Rouet P. et al., (1994). Mol Cell Biol, 14, 8096-106.
60. Sale, J.E. and Neuberger, M.S. (1998). Immunity, 9, 859-869.
61. Sandor, Z., et al., (2004). Nucleic Acids Res, 32, 1866-1873.
62. Shimizu, T. and Yamagishi, H. (1992). Embo J, 11, 4869-4875.
63. Soulas-Sprauel, P. et al., (2007). J Exp Med, 204, 1717-27.
64. Sur, M., et al., (2007). Mod Pathol, 20, 1113-1120.
65. Suzumiya, J., et al., (1997). J Pathol, 182, 86-91.
66. Weterings, E. and Chen, D.J. (2008). Cell Res, 18, 114-124.
67. Yan CT, et al., (2007). Nature 449, 478-82.
68.Zucman-Rossi et al., (1998) Proc Natl Acad Sci USA, 95, 11786-11791

## Claims

1. A method of generating *in vifro* junctional variability in the nucleotide sequence of a polynucleotide of interest present in a intrachromosomal substrate/context in a eukaryotic cell which is competent for canonical Non Homologous End Joining pathway (NHEJ) repair, comprising the steps of:
a) generating a double-strand break (DSB) in the DNA sequence of said polynucleotide, thereby providing broken ends in said polynucleotide in said eukaryotic cells,
b) providing the polymerase Terminal Deoxynucleotidyl Transferase (TdT) as a functional protein in the cells resulting from step a), in conditions enabling said TdT to add Non-templated nucleotides (N nucleotides) to the 3' ends of said broken ends before ligation of said ends through canonical Non Homologous End Joining pathway (NHEJ) thereby allowing a mutagenic repair to take place at the DSB site.

2. A method according to claim 1 wherein the double-strand break (DSB) is generated as (i) a targeted DSB in the DNA sequence of either a target polynucleotide or a random polynucleotide or as (ii) a random DSB in the DNA sequence of either a target polynucleotide or a random polynucleotide.

3. A method according to claim 1 or 2, wherein the DSB is generated by using a chemical reagent, a physical reagent, an enzyme or a combination thereof.

4. A method according to claim 1, 2 or 3, wherein the DSB is generated by cleavage with a nuclease, especially a meganuclease, in particular a meganuclease chosen among Homing Endonucleases (HEs), an artificial endonuclease such a Zinc Finger Nuclease, or an engineered endonuclease.

5. A method according to any of claims 1 to 4 wherein the meganuclease used for cleavage of the polynucleotide is chosen among:
- meganucleases which generate either 3' protruding ends in the broken junctions of the DSB or blunt ends in the broken junctions of the DSB;
- meganucleases which generate 5' protruding ends provided that it operates in conjunction with an enzyme which enables said 5' protruding ends to be modified into 3' protruding ends or into blunt ends.

6. A method according to any of claims 1 to 4, wherein the DSB is generated by cleavage of a polynucleotide with the I-Scel endonuclease provided said polynucleotide comprises one or more than one recognition site(s) for I-Scel, constituting the site(s) for the generation of the DSB.

7. A method according to any of claims 1 to 6, wherein the broken ends resulting from the DSB are obtained from a single event or from multiple events.

8. A method according to any of claims 1 to 7, wherein the DSB is carried out in a polynucleotide of interest having one or a combination of the following features:
- it is a nucleic acid naturally present in the eukaryotic cell wherein it is targeted or randomly considered or it is a derivative thereof or variant thereof;
- it is a nucleic acid which is heterologous to the chromosomal nucleic acid of the eukaryotic cell wherein it is targeted or randomly considered;
- it is a nucleic acid present as an insert into the chromosomal substrate of the cell wherein it is targeted or randomly considered, either as a result of a random insertion or as a result of targeted insertion;
- It is a modified nucleic acid with respect to its identified wild-type form;
- It is a nucleic acid of a gene or of a fragment of a gene, such as an expression regulatory sequence, in particular a promoter, a coding sequence, an exon, an intron, or it is a non coding sequence;
- It is a nucleic acid that originates from a eukaryotic cell or from a prokaryotic cell, including a pathogenic organism;
- It is a nucleic acid that is present either as a single copy or as multiple copies in the chromosomal substrate.

9. A method according to any of claims 1 to 8, wherein the DSB is generated in a polynucleotide, especially in a target polynucleotide wherein one or more than one nuclease, especially a meganuclease, cleavage site(s) has (have) been inserted or engineered.

10. A method according to any of claims 1 to 9, wherein the TdT is expressed transiently or in a regulated manner in the cells, especially after transfection or transduction of said cells with an expression vector comprising a transgene including the TdT coding sequence or after transfection or transduction with the RNA transcript of a TdT gene or wherein the TdT is delivered to the cell as a functional protein.

11. A method according to any of claims 1 to 10, wherein the nuclease, especially the meganuclease, is expressed transiently or in a regulated manner in the cells after transfection or transduction of said cells with an expression vector comprising a transgene including its coding sequence, or after transfection or transduction with the RNA transcript of a nuclease, especially a meganuclease, gene or wherein the nuclease, especially a meganuclease, is delivered to the cell as a functional protein.

12. A method according to any of claims 1 to 11, wherein the junctional variability results from an overall number of added and deleted nucleotides which is conservative.

13. A method according to any of claims 1 to 12, wherein the junctional variability is conservative in a window of about 100 nucleotides and up to about 300 nucleotides around the DSB, or in a window of about 100 nucleotides and up to 300 nucleotides beginning at the ends of the broken junction resulting from the cleavage at the level of the DSB.

14. A method according to any of claims 1 to 13, wherein the eukaryotic cell does not naturally express functional Terminal Deoxynucleotidyl Transferase (TaT).

15. A method according to any of claims 1 to 14, wherein the eukaryotic cells are chosen among cultured cells, primary cells, secondary cells, cell lines, stem cells, progenitor cells and differentiated tissues, including such cells or tissues that are mutated and/or naturally deficient or rendered deficient in at least a second nucleic acid of interest, especially a gene, to the exclusion of human embryonic stem cells obtained through embryo destruction.

16. A method according to any of claims 1 to 15, wherein the eukaryotic cells are mammalian cells in particular human cells, or murine cells, bird cells, fish cells, yeast cells or fungi or are plant cells, to the exclusion of human embryonic stem cells obtained through embryo destruction.

17. A method according to any of claims 1 to 16, wherein the polynucleotide of interest in the chromosomal context is contained in a gene, especially in a coding sequence, or is contained in a regulatory sequence such as a promoter, or is contained in a post translational active sequence.

18. A method for creating junctional variability in the nucleotide sequence of a target polynucleotide comprising:
a) implementing the method of any of claims 1 to 17 on a polynucleotide of interest;
b) recovering cells comprising the polynucleotide of interest which has been mutated and repaired as a result of said method and optionally recovering said mutated repaired polynucleotide of interest.

19. A method of any of claims 1 to 18, wherein the polynucleotide of interest is selected among:
- - a gene expressing an enzyme, such as a kinase, in particular wherein the sequence of the polynucleotide of interest encodes the active site of the enzyme,
- a gene expressing a cell receptor,
- a gene expressing a structural protein, a secreted protein, or a regulatory protein, such as an interleukin or an interferon,
- a polynucleotide, especially a gene, of a virus, a bacterium or a parasite,
- regulatory sequences for transcription or for expression of said genes.

20. A method for determining occurrence(s) of generation of double strand break(s) in a cell, or in a population of cells, comprising the steps of:
a) performing the method defined in any one of claims 1 to 19 on said cell,
b) evaluating the junctional variability generated in said cell.

21. Use of TdT as a marker of DSB events wherein a DSB is repaired in a way generating junctional variability at the locus of said DSB.

## Patentansprüche

1. Verfahren zum Erzeugen einer in vitro junktionalen Variabilität in der Nukleotidsequenz eines interessanten Polynukleotids, das in einem intrachromosomalen Substrat/Kontext in einer eukaryontischen Zelle vorliegt, die zur Reparatur des kanonischen Wegs der nicht-homologen Endverknüpfung (NHEJ) fähig ist, umfassend die Schritte:
a) Erzeugen eines Doppelstrangbruchs (DSB) in der DNA-Sequenz des Polynukleotids, wodurch in dem Polynukleotid in den eukaryontischen Zellen gebrochene Enden vorgesehen werden,
b) Vorsehen der Polymerase Terminale-Desoxynukleotidyl-Transferase (TdT) als funktionelles Protein in den Zellen, resultierend aus Schritt a), unter Bedingungen, die es der TdT ermöglichen, Nicht-Template-Nukleotide (N-Nukleotide) an die 3'-Enden der gebrochenen Enden vor einer Ligation der Enden durch den kanonischen Weg der nicht-homologen Endverknüpfung (NHEJ) hinzuzufügen, um zu ermöglichen, dass eine mutagene Reparatur an der DSB-Stelle stattfindet.

2. Verfahren nach Anspruch 1, wobei der Doppelstrangbruch (DSB) als (i) gezielter DSB entweder in der DNA-Sequenz eines Zielpolynukleotids oder eines Zufallspolynukleotids oder als (ii) zufälliger DSB entweder in der DNA-Sequenz eines Zielpolynukleotids oder eines Zufallspolynukleotids erzeugt wird.

3. Verfahren nach Anspruch 1 oder 2, wobei der DSB dadurch erzeugt wird, dass ein chemisches Reagens, ein physikalisches Reagens, ein Enzym oder eine Kombination davon verwendet wird.

4. Verfahren nach Anspruch 1, 2 oder 3, wobei der DSB durch Spalten mit einer Nuklease, speziell einer Meganuklease, insbesondere einer Meganuklease, die aus Homing-Endonukleasen (HEs), einer künstlichen Endonuklease, wie beispielsweise einer Zinkfingernuklease, oder einer manipulierten Endonuklease, erzeugt wird.

5. Verfahren nach einem der Ansprüche 1 bis 4, wobei die Meganuklease, die zur Spaltung des Polynukleotids verwendet wird, ausgewählt wird aus:
- Meganukleasen, die entweder überhängende 3'-Enden in den gebrochenen Verknüpfungen des DSB oder glatte Enden in den gebrochenen Verknüpfungen des DSB erzeugen;
- Meganukleasen, die überhängende 5'-Enden erzeugen, vorausgesetzt, dass sie zusammen mit einem Enzym operieren, welches es möglich macht, dass die überhängenden 5'-Enden zu überhängenden 3'-Enden oder zu glatten Enden modifiziert werden.

6. Verfahren nach einem der Ansprüche 1 bis 4, wobei der DSB durch Spalten eines Polynukleotids mit der I-SceI Endonuklease erzeugt wird, vorausgesetzt, dass das Polynukleotid eine oder mehr als eine Erkennungsstelle(n) für I-SceI umfasst, die die Stelle(n) zur Erzeugung des DSB bildet/bilden.

7. Verfahren nach einem der Ansprüche 1 bis 6, wobei die gebrochenen Enden, die aus dem DSB resultieren, aus einem einzelnen Ereignis oder aus mehreren Ereignissen erhalten werden.

8. Verfahren nach einem der Ansprüche 1 bis 7, wobei der DSB in einem interessanten Polynukleotid mit einem oder einer Kombination der folgenden Merkmale durchgeführt wird:
- es handelt sich um eine Nukleinsäure, die natürlicherweise in der eukaryontischen Zelle vorkommt, wobei sie als Ziel gesetzt oder zufällig berücksichtigt wird oder sie ein Derivat davon oder Variante davon ist;
- es handelt sich um eine Nukleinsäure, die zur chromosomalen Nukleinsäure der eukaryontischen Zelle heterolog ist, wobei sie als Ziel gesetzt oder willkürlich berücksichtigt wird;
- es handelt sich um eine Nukleinsäure, die als Insert in dem chromosomalen Substrat der Zelle vorliegt, wobei sie als Ziel gesetzt oder willkürlich berücksichtigt wird, entweder als Ergebnis einer zufälligen Insertion oder als Ergebnis einer gezielten Insertion;
- es handelt sich um eine modifizierte Nukleinsäure im Hinblick auf die identifizierte Wildtypform;
- es handelt sich um eine Nukleinsäure oder ein Gen oder ein Genfragment, wie beispielsweise eine expressionsregulatorische Sequenz, insbesondere einen Promotor, eine Kodierungssequenz, ein Exon, ein Intron oder um eine nicht kodierende Sequenz;
- es handelt sich um eine Nukleinsäure, die von einer eukaryontischen Zelle oder von einer prokaryontischen Zelle, einschließlich einem pathogenen Organismus, stammt;
- es handelt sich um eine Nukleinsäure, die entweder als einzelne Kopie oder als mehrere Kopien in dem chromosomalen Substrat vorliegt.

9. Verfahren nach einem der Ansprüche 1 bis 8, wobei der DSB in einem Polynukleotid, insbesondere in einem Zielpolynukleotid, erzeugt wird, wobei durch eine oder mehr als eine Nuklease, insbesondere eine Meganuklease, Spaltstelle(n) insertiert oder eingearbeitet wurde(n).

10. Verfahren nach einem der Ansprüche 1 bis 9, wobei die TdT transient oder reguliert in den Zellen exprimiert wird, insbesondere nach einer Transfektion oder Transduktion der Zellen mit einem Expressionsvektor, umfassend ein Transgen mit der TdT-Kodierungssequenz, oder nach einer Transfektion oder Transduktion mit dem RNA-Transkript eines TdT-Gens, oder wobei die TdT an die Zelle als funktionelles Protein abgegeben wird.

11. Verfahren nach einem der Ansprüche 1 bis 10, wobei die Nuklease, insbesondere die Meganuklease, transient oder reguliert in den Zellen nach einer Transfektion oder Transduktion der Zellen mit einem Expressionsvektor, umfassend ein Transgen mit seiner Kodierungssequenz, oder nach einer Transfektion oder Transduktion mit dem RNA-Transkript eines Nuklease-, insbesondere eines Meganuklease-, Gens exprimiert wird oder wobei die Nuklease, insbesondere eine Meganuklease, als funktionelles Protein an die Zelle abgegeben wird.

12. Verfahren nach einem der Ansprüche 1 bis 11, wobei die junktionale Variabilität aus einer Gesamtzahl von hinzugefügten oder deletierten Nukleotiden resultiert, die konservativ ist.

13. Verfahren nach einem der Ansprüche 1 bis 12, wobei die junktionale Variabilität in einem Fenster von etwa 100 Nukleotiden und bis zu etwa 300 Nukleotiden um den DSB oder in einem Fenster von etwa 100 Nukleotiden und bis zu 300 Nukleotiden, beginnend an den Enden der gebrochenen Verknüpfung, die von der Spaltung auf der Ebene des DSB resultiert, konservativ ist.

14. Verfahren nach einem der Ansprüche 1 bis 13, wobei die eukaryontische Zelle die funktionelle Terminale-Desoxynukleotidyl-Transferase (TdT) nicht natürlicherweise exprimiert.

15. Verfahren nach einem der Ansprüche 1 bis 14, wobei die eukaryontischen Zellen ausgewählt werden aus kultivierten Zellen, primären Zellen, sekundären Zellen, Zelllinien, Stammzellen, Progenitorzellen und differenzierten Geweben, einschließlich solchen Zellen oder Geweben, die mutiert und/oder natürlicherweise fehlerhaft sind oder in mindestens einer zweiten interessanten Nukleinsäure, insbesondere einem Gen, unter Ausschluss von menschlichen embryonalen, durch die Zerstörung eines Embryos erhaltenen Stammzellen fehlerhaft gemacht werden.

16. Verfahren nach einem der Ansprüche 1 bis 15, wobei die eukaryontischen Zellen Säugetierzellen, insbesondere menschliche Zellen, oder Mauszellen, Vogelzellen, Fischzellen, Hefezellen oder Pilze sind oder Pflanzenzellen sind, unter Ausschluss der menschlichen embryonalen Stammzellen, die durch eine Embryozerstörung erhalten werden.

17. Verfahren nach einem der Ansprüche 1 bis 16, wobei das interessante Polynukleotid im chromosomalen Kontext in einem Gen, insbesondere in einer Kodierungssequenz, enthalten ist oder in einer regulatorischen Sequenz, wie beispielsweise einem Promotor, enthalten ist oder in einer posttranslationalen aktiven Sequenz enthalten ist.

18. Verfahren zum Erzeugen einer junktionalen Variabilität in der Nukleotidsequenz eines Zielpolynukleotids, umfassend:
a) Ausführen des Verfahrens nach einem der Ansprüche 1 bis 17 an einem interessanten Polynukleotid;
b) Gewinnen von Zellen, umfassend das interessante Polynukleotid, das als Ergebnis des Verfahrens mutiert und repariert wurde, und wahlweises Gewinnen des mutierten reparierten interessanten Polynukleotids.

19. Verfahren nach einem der Ansprüche 1 bis 18, wobei das interessante Polynukleotid ausgewählt ist aus:
- einem Gen, das ein Enzym, wie beispielsweise eine Kinase, exprimiert, wobei insbesondere die Sequenz des interessanten Polynukleotids die aktive Stelle des Enzymes kodiert,
- einem Gen, das einen Zellrezeptor exprimiert,
- einem Gen, das ein Strukturprotein, ein sekretiertes Protein oder ein regulatorisches Protein, wie beispielsweise ein Interleukin oder ein Interferon, exprimiert,
- einem Polynukleotid, insbesondere einem Gen, eines Virus, eines Bakteriums oder eines Parasiten,
- regulatorischen Sequenzen zur Transkription oder zur Expression der Gene.

20. Verfahren zum Bestimmen des Auftretens des Erzeugens eines Doppelstrangbruchs/von Doppelstrangbrüchen in einer Zelle oder in einer Zellpopulation, umfassend die Schritte:
a) Durchführen des Verfahrens nach einem der Ansprüche 1 bis 19 an der Zelle,
b) Bewerten der junktionalen Variabilität, die in der Zelle erzeugt wird.

21. Verwenden von TdT als Marker von DSB-Ereignissen, wobei ein DSB auf eine Weise repariert wird, die eine junktionale Variabilität am Ort des DSB erzeugt.

## Revendications

1. Procédé de génération *in vitro* d'une variabilité jonctionnelle dans la séquence nucléotidique d'un polynucléotide d'intérêt présent dans un substrat/contexte intrachromosomique dans une cellule eucaryote qui est compétente pour la réparation par la voie canonique de ligature d'extrémités non homologues (NHEJ), comprenant les étapes de :
a) génération d'une cassure double-brin (DSB) dans la séquence d'ADN dudit polynucléotide, fournissant ainsi des extrémités cassées dans ledit polynucléotide dans lesdites cellules eucaryotes,
b) fourniture de la polymérase terminale désoxynucléotidyle transférase (TdT) en tant que protéine fonctionnelle dans les cellules résultant de l'étape a), dans des conditions permettant à ladite TdT d'ajouter des nucléotides aléatoires (nucléotides N) aux extrémités 3' desdites extrémités cassées avant la ligature desdites extrémités par la voie canonique de ligature d'extrémités non homologues (NHEJ), permettant ainsi à une réparation mutagène d'avoir lieu au niveau du site de la DSB.

2. Procédé selon la revendication 1, dans lequel la cassure double-brin (DSB) est générée comme (i) une DSB ciblée dans la séquence d'ADN soit d'un polynucléotide cible soit d'un polynucléotide aléatoire ou comme (ii) une DSB aléatoire dans la séquence d'ADN soit d'un polynucléotide cible soit d'un polynucléotide aléatoire.

3. Procédé selon la revendication 1 ou 2, dans lequel la DSB est générée en utilisant un réactif chimique, un réactif physique, une enzyme ou une combinaison de ceux-ci.

4. Procédé selon la revendication 1, 2 ou 3, dans lequel la DSB est généré par clivage avec une nucléase, notamment une méganucléase, en particulier une méganucléase choisie parmi les endonucléases homing (HEs), une endonucléase artificielle telle qu'une nucléase à doigt de zinc, ou une endonucléase spécialement construite.

5. Procédé selon l'une quelconque des revendications 1 à 4, dans lequel la méganucléase utilisée pour le clivage du polynucléotide est choisie parmi :
- les méganucléases qui génèrent soit des extrémités saillantes 3' dans les jonctions cassées de la DSB soit des extrémités franches dans les jonctions cassées de la DSB ;
- les méganucléases qui génèrent des extrémités saillantes 5' à condition qu'elle fonctionne en conjonction avec une enzyme qui permet auxdites extrémités saillantes 5' d'être modifiées en extrémités saillantes 3' ou en extrémités franches.

6. Procédé selon l'une quelconque des revendications 1 à 4, dans lequel la DSB est générée par clivage d'un polynucléotide avec l'endonucléase I-Scel à condition que ledit polynucléotide comprenne un ou plus d'un site(s) de reconnaissance pour I-Scel, constituant le(s) site(s) pour la génération de la DSB.

7. Procédé selon l'une quelconque des revendications 1 à 6, dans lequel les extrémités cassées résultant de la DSB sont obtenues à partir d'un unique événement ou de plusieurs événements.

8. Procédé selon l'une quelconque des revendications 1 à 7, dans lequel la DSB est effectuée dans un polynucléotide d'intérêt ayant une ou une combinaison des caractéristiques suivantes :
- il s'agit d'un acide nucléique présent naturellement dans la cellule eucaryote dans laquelle il est ciblé ou considéré de façon aléatoire ou il s'agit d'un dérivé de celui-ci ou d'un variant de celui-ci ;
- il s'agit d'un acide nucléique qui est hétérologue à l'acide nucléique chromosomique de la cellule eucaryote dans laquelle il est ciblé ou considéré de façon aléatoire ;
- il s'agit d'un acide nucléique présent sous la forme d'un insert dans le substrat chromosomique de la cellule dans laquelle il est ciblé ou considéré de façon aléatoire, soit en résultat d'une insertion aléatoire soit en résultat d'une insertion ciblée ;
- il s'agit d'un acide nucléique modifié par rapport à sa forme de type sauvage identifiée ;
- il s'agit d'un acide nucléique d'un gène ou d'un fragment d'un gène, comme une séquence régulatrice d'expression, en particulier un promoteur, une séquence codante, un exon, un intron, ou il s'agit d'une séquence non codante ;
- il s'agit d'un acide nucléique qui provient d'une cellule eucaryote ou d'une cellule procaryote, y compris un organisme pathogène ;
- il s'agit d'un acide nucléique qui est présent soit en une seule copie soit en plusieurs copies dans le substrat chromosomique.

9. Procédé selon l'une quelconque des revendications 1 à 8, dans lequel la DSB est générée dans un polynucléotide, notamment dans un polynucléotide cible dans lequel un ou plus d'un site(s) de clivage par une nucléase, notamment une méganucléase, a(ont) été inséré(s) ou spécialement construite (s).

10. Procédé selon l'une quelconque des revendications 1 à 9, dans lequel la TdT est exprimée de manière transitoire ou de manière régulée dans les cellules, notamment après la transfection ou la transduction desdites cellules avec un vecteur d'expression comprenant un transgène incluant la séquence codant pour la TdT ou après la transfection ou la transduction avec le transcript d'ARN d'un gène de TdT, ou dans lequel la TdT est délivrée à la cellule sous forme d'une protéine fonctionnelle.

11. Procédé selon l'une quelconque des revendications 1 à 10, dans lequel la nucléase, notamment la méganucléase, est exprimée de manière transitoire ou de manière régulée dans les cellules après la transfection ou la transduction desdites cellules avec un vecteur d'expression comprenant un transgène incluant sa séquence codante, ou après la transfection ou la transduction avec le transcript d'ARN d'un gène de nucléase, notamment d'une méganucléase, ou dans lequel la nucléase, notamment une méganucléase, est délivrée à la cellule sous la forme d'une protéine fonctionnelle.

12. Procédé selon l'une quelconque des revendications 1 à 11, dans lequel la variabilité jonctionnelle résulte d'un nombre global de nucléotides ajoutés et délétés qui soit conservatif.

13. Procédé selon l'une quelconque des revendications 1 à 12, dans lequel la variabilité jonctionnelle est conservatrice dans une fenêtre d'environ 100 nucléotides et jusqu'à environ 300 nucléotides autour de la DSB, ou dans une fenêtre d'environ 100 nucléotides et jusqu'à 300 nucléotides en commençant aux extrémités de la jonction cassée résultant du clivage au niveau de la DSB.

14. Procédé selon l'une quelconque des revendications 1 à 13, dans lequel la cellule eucaryote n'exprime pas naturellement de la terminale désoxynucléotidyle transférase (TdT) fonctionnelle.

15. Procédé selon l'une quelconque des revendications 1 à 14, dans lequel les cellules eucaryotes sont choisies parmi des cellules en culture, des cellules primaires, des cellules secondaires, des lignées cellulaires, des cellules souches, des cellules progénitrices et des tissus différenciés, y compris ces cellules ou tissus qui sont mutés et/ou naturellement déficients ou rendus déficients en au moins un deuxième acide nucléique d'intérêt, en particulier un gène, à l'exclusion des cellules souches embryonnaires humaines obtenues par destruction d'embryon.

16. Procédé selon l'une quelconque des revendications 1 à 15, dans lequel les cellules eucaryotes sont des cellules de mammifère en particulier des cellules humaines ou des cellules murines, des cellules d'oiseaux, des cellules de poisson, des cellules de levures ou de champignons ou sont des cellules végétales, à l'exclusion des cellules souches embryonnaires humaines obtenues par destruction d'embryon.

17. Procédé selon l'une quelconque des revendications 1 à 16, dans lequel le polynucléotide d'intérêt dans le contexte chromosomique est contenu dans un gène, en particulier dans une séquence codante, ou est contenu dans une séquence régulatrice telle qu'un promoteur, ou est contenu dans une séquence active post traductionnelle.

18. Procédé pour la création d'une variabilité jonctionnelle dans la séquence nucléotidique d'un polynucléotide cible comprenant :
a) la mise en oeuvre du procédé selon l'une quelconque des revendications 1 à 17 sur un polynucléotide d'intérêt ;
b) la récupération des cellules comprenant le polynucléotide d'intérêt, qui a été muté et réparé en résultat dudit procédé et éventuellement la récupération dudit polynucléotide muté réparé d'intérêt.

19. Procédé selon l'une quelconque des revendications 1 à 18, dans lequel le polynucléotide d'intérêt est choisi parmi :
- un gène exprimant une enzyme, telle qu'une kinase, en particulier dans lequel la séquence du polynucléotide d'intérêt code pour le site actif de l'enzyme,
- un gène exprimant un récepteur cellulaire,
- un gène exprimant une protéine de structure, une protéine sécrétée, ou une protéine de régulation, telle qu'une interleukine ou un interféron,
- un polynucléotide, notamment un gène, d'un virus, d'une bactérie ou d'un parasite,
- des séquences régulatrices pour la transcription ou pour l'expression desdits gènes.

20. Procédé pour la détermination de l'occurrence(des occurrences) de génération de cassure(s) double-brin dans une cellule, ou dans une population de cellules, comprenant les étapes de :
a) réalisation du procédé défini selon l'une quelconque des revendications 1 à 19 sur ladite cellule,
b) évaluation de la variabilité jonctionnelle générée dans ladite cellule.

21. Utilisation de TdT comme marqueur d'événements DSB dans laquelle une DSB est réparée d'une façon générant une variabilité jonctionnelle au niveau du locus de ladite DSB.


### SEQUENCE LISTING

<110> INSTITUT PASTEUR
<120> USE OF TERMINAL DEOXYNUCLEOTIDYL TRANSFERASE FOR MUTAGENIC DNA REPAIR TO GENERATE VARIABILITY, AT A DETERMINED POSITION IN DNA.
<130> B08392 AD/AFL/CAL
<160> 113
<170> PatentIn version 3.3
<210> 1
   <211> 51
   <212> DNA
   <213> Artificial
<220>
   <223> mutated fragment of a clone
<400> 1
   tctagagcaa cacggaagga attaccctgt tatccctatc tagatatgaa a 51
<210> 2
   <211> 50
   <212> DNA
   <213> Artificial
<220>
   <223> mutated fragment of a clone
<400> 2
   tctagagcaa cacggaagga attaccctgt tatcctatct agatatgaaa 50
<210> 3
   <211> 45
   <212> DNA
   <213> Artificial
<220>
   <223> mutated fragment of a clone
<400> 3
   tctagagcaa cacggaagga attaccctgt tatctagata tgaaa 45
<210> 4
   <211> 43
   <212> DNA
   <213> artificial
<220>
   <223> mutated fragment of a clone
<400> 4
   tctagagcaa cacggaagga attaccccta tctagatatg aaa 43
<210> 5
   <211> 43
   <212> DNA
   <213> Artificial
<220>
   <223> mutated fragment of a clone
<400> 5
   tctagagcaa cacggaagga attatcccta tctagatatg aaa 43
<210> 6
   <211> 42
   <212> DNA
   <213> Artificial
<220>
   <223> mutated fragment of a clone
<400> 6
   tctagagcaa cacggaagga attaccctgt ctagatatga aa 42
<210> 7
   <211> 42
   <212> DNA
   <213> Artificial
<220>
   <223> mutated fragment of a clone
<400> 7
   tctagagcaa cacggaagga attaccctat ctagatatga aa 42
<210> 8
   <211> 35
   <212> DNA
   <213> Artificial
<220>
   <223> mutated fragment of a clone
<400> 8
   tctagagcaa cacggaagga attctagata tgaaa 35
<210> 9
   <211> 41
   <212> DNA
   <213> Artificial
<220>
   <223> mutated fragment of a clone
<220>
   <221> misc_feature
   <222> (29)..(30)
   <223> Deletion of 23 nucleotides
<400> 9
   tctagagcaa cacggaagga attaccctgc acgccatgta g 41
<210> 10
   <211> 40
   <212> DNA
   <213> Artificial
<220>
   <223> mutated fragment of a clone
<220>
   <221> misc_feature
   <222> (23)..(24)
   <223> deletion of 32 nucleotides
<400> 10
   gagacccaag ctggctagcg ctccctatct agatatgaaa 40
<210> 11
   <211> 43
   <212> DNA
   <213> Artificial
<220>
   <223> mutated fragment of a clone
<220>
   <221> misc_feature
   <222> (21)..(22)
   <223> deletion of 55 nucleotides
<400> 11
   tctagagcaa cacggaagga attccttgcg gtccgaatgg gcc 43
<210> 12
   <211> 39
   <212> DNA
   <213> Artificial
<220>
   <223> mutated fragment of a clone
<220>
   <221> misc_feature
   <222> (15)..(16)
   <223> Deletion of 67 nucleotides
<400> 12
   tataggggga cccaagccat gtagtgtatt gaccgattc 39
<210> 13
   <211> 44
   <212> DNA
   <213> Artificial
<220>
   <223> mutated fragment of a clone
<220>
   <221> misc_feature
   <222> (18)..(19)
   <223> Deletion of 188 nucleotides
<400> 13
   tctagagcaa cacggaagct cagattccca accaacaaga gctc 44
<210> 14
   <211> 34
   <212> DNA
   <213> Artificial
<220>
   <223> mutated fragment of a clone
<220>
   <221> misc_feature
   <222> (29)..(31)
   <223> Deletion of 18 nucleotides + addition of 1 nucleotide
<400> 14
   tctagagcaa cacggaagga attaccctgc gaaa 34
<210> 15
   <211> 51
   <212> DNA
   <213> Artificial
<220>
   <223> mutated fragment of a clone
<400> 15
   tctagagcaa cacggaagga attaccctgt tatccctatc tagatatgaa a 51
<210> 16
   <211> 45
   <212> DNA
   <213> Artificial
<220>
   <223> mutated fragment of a clone
<400> 16
   tctagagcaa cacggaagga attaccctgt tatctagata tgaaa 45
<210> 17
   <211> 43
   <212> DNA
   <213> Artificial
<220>
   <223> mutated fragment of a clone
<400> 17
   tctagagcaa cacggaagga attaccctgt tatagatatg aaa 43
<210> 18
   <211> 52
   <212> DNA
   <213> Artificial
<220>
   <223> mutated fragment of a clone
<400> 18
   agatatgaaa tctagagcaa cacggaagga attaccctat ctagatatga aa 52
<210> 19
   <211> 36
   <212> DNA
   <213> Artificial
<220>
   <223> mutated fragment of a clone
<400> 19
   tctagagcaa cacggaagga ctatctagat atgaaa 36
<210> 20
   <211> 53
   <212> DNA
   <213> Artificial
<220>
   <223> mutated fragment of a clone
<220>
   <221> misc_feature
   <222> (31)..(36)
   <223> Addition of 4 nucleotides
<400> 20
   tctagagcaa cacggaagga attaccctgt tttatcccta tctagatatg aaa 53
<210> 21
   <211> 30
   <212> DNA
   <213> Artificial
<220>
   <223> mutated fragment of a clone
<220>
   <221> misc_feature
   <222> (10)..(12)
   <223> Deletion of 34 nucleotides + addition of 1 nucleotide
<400> 21
   gctggctagc ctccctatct agatatgaaa 30
<210> 22
   <211> 40
   <212> DNA
   <213> Artificial
<220>
   <223> mutated fragment of a clone
<220>
   <221> misc_feature
   <222> (16)..(18)
   <223> Deletion of 55 nucleotides + addition of 1 nucleotide
<400> 22
   gctggctagc gctctattgt agtgtattga ccgattcctt 40
<210> 23
   <211> 52
   <212> DNA
   <213> Artificial
<220>
   <223> mutated fragment of a clone
<220>
   <221> misc_feature
   <222> (33)..(36)
   <223> Addition of 2 nucleotides
<400> 23
   tctagagcaa cacggaagga attaccctgt tatttcctat ctagatatga aa 52
<210> 24
   <211> 47
   <212> DNA
   <213> Artificial
<220>
   <223> mutated fragment of a clone
<220>
   <221> misc_feature
   <222> (27)..(30)
   <223> Addition of 2 nucleotides
<400> 24
   tctagagcaa cacggaagga attaccccgc cctatctaga tatgaaa 47
<210> 25
   <211> 51
   <212> DNA
   <213> Artificial
<220>
   <223> mutated fragment of a clone
<220>
   <221> misc_feature
   <222> (31)..(34)
   <223> Addition of 3 nucleotides
<400> 25
   tctagagcaa cacggaagga attaccctgt aggccctatc tagatatgaa a 51
<210> 26
   <211> 45
   <212> DNA
   <213> Artificial
<220>
   <223> mutated fragment of a clone
<220>
   <221> misc_feature
   <222> (33)..(39)
   <223> Addition of 5 nucleotides
<400> 26
   tctagagcaa cacggaagga attaccctgt tatgagggta tgaaa 45
<210> 27
   <211> 54
   <212> DNA
   <213> Artificial
<220>
   <223> mutated fragment of a clone
<220>
   <221> misc_feature
   <222> (30)..(37)
   <223> Addition of 6 nucleotides
<400> 27
   tctagagcaa cacggaagga attaccctgt ccttatccct atctagatat gaaa 54
<210> 28
   <211> 58
   <212> DNA
   <213> Artificial
<220>
   <223> mutated fragment of a clone
<220>
   <221> misc_feature
   <222> (32)..(41)
   <223> Addition of 8 nucleotides
<400> 28
   tctagagcaa cacggaagga attaccctgt tacaggttat ccctatctag atatgaaa 58
<210> 29
   <211> 56
   <212> DNA
   <213> Artificial
<220>
   <223> mutated fragment of a clone
<220>
   <221> misc_feature
   <222> (28)..(38)
   <223> Addition of 9 nucleotides
<400> 29
   tctagagcaa cacggaagga attaccctgg tatcttatcc ctatctagat atgaaa 56
<210> 30
   <211> 37
   <212> DNA
   <213> Artificial
<220>
   <223> mutated fragment of a clone
<220>
   <221> misc_feature
   <222> (10)..(20)
   <223> Deletion 26 + addition (9 bp)
<220>
   <221> misc-feature
   <222> (10)..(20)
   <223> Deletion of 26 nucleotides + addition of 9 nucleotides
<400> 30
   gctggctagc tagggttatc cctatctaga tatgaaa 37
<210> 31
   <211> 52
   <212> DNA
   <213> Artificial
<220>
   <223> mutated fragment of a clone
<220>
   <221> misc_feature
   <222> (33)..(35)
   <223> Addition of 1 nucleotide
<400> 31
   tctagagcaa cacggaagga attaccctgt tatcccctat ctagatatga aa 52
<210> 32
   <211> 53
   <212> DNA
   <213> Artificial
<220>
   <223> mutated fragment of a clone
<220>
   <221> misc_feature
   <222> (33)..(36)
   <223> Addition of 2 nucleotides
<220>
   <221> misc_feature
   <222> (34)..(36)
   <223> Addition (2 bp)
<400> 32
   tctagagcaa cacggaagga attaccctgt tataccccta tctagatatg aaa 53
<210> 33
   <211> 53
   <212> DNA
   <213> Artificial
<220>
   <223> mutated fragment of a clone
<220>
   <221> misc_feature
   <222> (33)..(36)
   <223> Addition of 2 nucleotides
<400> 33
   tctagagcaa cacggaagga attaccctgt tatttcccta tctagatatg aaa 53
<210> 34
   <211> 53
   <212> DNA
   <213> Artificial
<220>
   <223> mutated fragment of a clone
<220>
   <221> misc_feature
   <222> (33)..(36)
   <223> Addition of 2 nucleotides
<400> 34
   tctagagcaa cacggaagga attaccctgt tatatcccta tctagatatg aaa 53
<210> 35
   <211> 54
   <212> DNA
   <213> Artificial
<220>
   <223> mutated fragment of a clone
<220>
   <221> misc_feature
   <222> (33)..(37)
   <223> Addition of 3 nucleotides
<400> 35
   tctagagcaa cacggaagga attaccctgt tatagtccct atctagatat gaaa 54
<210> 36
   <211> 55
   <212> DNA
   <213> Artificial
<220>
   <223> mutated fragment of a clone
<220>
   <221> misc_feature
   <222> (33)..(38)
   <223> Addition of 4 nucleotides
<400> 36
   tctagagcaa cacggaagga attaccctgt tatagggccc tatctagata tgaaa 55
<210> 37
   <211> 56
   <212> DNA
   <213> Artificial
<220>
   <223> mutated fragment of a clone
<220>
   <221> misc_feature
   <222> (33)..(39)
   <223> Addition of 5 nucleotides
<400> 37
   tctagagcaa cacggaagga attaccctgt tatcctttcc ctatctagat atgaaa 56
<210> 38
   <211> 60
   <212> DNA
   <213> Artificial
<220>
   <223> mutated fragment of a clone
<220>
   <221> misc_feature
   <222> (33)..(43)
   <223> Addition of 9 nucleotides
<220>
   <221> misc_feature
   <222> (34)..(42)
   <223> Addition (9 bp)
<400> 38
   tctagagcaa cacggaagga attaccctgt tatttccttt atccctatct agatatgaaa 60
<210> 39
   <211> 43
   <212> DNA
   <213> Artificial
<220>
   <223> mutated fragment of a clone
<400> 39
   tctagagcaa cacggaagga attaccccta tctagatatg aaa 43
<210> 40
   <211> 42
   <212> DNA
   <213> Artificial
<220>
   <223> mutated fragment of a clone
<400> 40
   tctagagcaa cacggaagga attaccctgt ctagatatga aa 42
<210> 41
   <211> 42
   <212> DNA
   <213> Artificial
<220>
   <223> mutated fragment of a clone
<400> 41
   tctagagcaa cacggaagga attaccctat ctagatatga aa 42.
<210> 42
   <211> 40
   <212> DNA
   <213> Artificial
<220>
   <223> mutated fragment of a clone
<400> 42
   tctagagcaa cacggaagga atccctatct agatatgaaa 40
<210> 43
   <211> 36
   <212> DNA
   <213> Artificial
<220>
   <223> mutated fragment of a clone
<400> 43
   tctagagcaa cacggaagcc ctatctagat atgaaa 36
<210> 44
   <211> 34
   <212> DNA
   <213> Artificial
<220>
   <223> mutated fragment of a clone
<400> 44
   tctagagcaa cacggaagga atctagatat gaaa 34
<210> 45
   <211> 37
   <212> DNA
   <213> Artificial
<220>
   <223> mutated fragment of a clone
<220>
   <221> misc_feature
   <222> (23)..(24)
   <223> Deletion of 55 nucleotides
<400> 45
   tctagagcaa cacggaagga attccttgcg gtccgaa 37
<210> 46
   <211> 33
   <212> DNA
   <213> Artificial
<220>
   <223> mutated fragment of a clone
<220>
   <221> misc_feature
   <222> (14)..(16)
   <223> Deletion 38 + addition (1 bp)
<220>
   <221> misc_feature
   <222> (14)..(16)
   <223> Deletion of 38 nucleotides + addition of 1 nucleotide
<400> 46
   gacccaagct ggctccccta tctagatatg aaa 33
<210> 47
   <211> 42
   <212> DNA
   <213> Artificial
<220>
   <223> mutated fragment of a clone
<400> 47
   tctagagcaa cacggaagga attaccctgt ctagatatga aa 42
<210> 48
   <211> 39
   <212> DNA
   <213> Artificial
<220>
   <223> mutated fragment of a clone
<400> 48
   tctagagcaa cacggaagga attaccctga tcacgccat 39
<210> 49
   <211> 28
   <212> DNA
   <213> Artificial
<220>
   <223> mutated fragment of a clone
<220>
   <221> misc_feature
   <222> (18)..(19)
   <223> Deletion of 32 nucleotides
<400> 49
   tctagagcaa cacggaagat cacgccat 28
<210> 50
   <211> 31
   <212> DNA
   <213> Artificial
<220>
   <223> mutated fragment of a clone
<220>
   <221> misc_feature
   <222> (14)..(15)
   <223> Deletion 48
<220>
   <221> misc_feature
   <222> (14)..(15)
   <223> Deletion of 48 nucleotides
<400> 50
   ctatagggag accccctatc tagatatgaa a 31
<210> 51
   <211> 38
   <212> DNA
   <213> Artificial
<220>
   <223> mutated fragment of a clone
<220>
   <221> misc_feature
   <222> (23)..(24)
   <223> Deletion of 55 nucleotides
<400> 51
   tctagagcaa cacggaagga attccttgcg gtccgaat 38
<210> 52
   <211> 30
   <212> DNA
   <213> Artificial
<220>
   <223> mutated fragment of a clone
<220>
   <221> misc_feature
   <222> (15)..(16)
   <223> Deletion 67
<220>
   <221> misc_feature
   <222> (15)..(16)
   <223> Deletion of 67 nucleotides
<400> 52
   ctatagggag acccacgcca tgtagtgtat 30
<210> 53
   <211> 34
   <212> DNA
   <213> Artificial
<220>
   <223> mutated fragment of a clone
<220>
   <221> misc_feature
   <222> (20)..(21)
   <223> Deletion of 163 nucleotides
<400> 53
   tctagagcaa cacggaagga gagtgaagaa ggac 34
<210> 54
   <211> 33
   <212> DNA
   <213> Artificial
<220>
   <223> mutated fragment of a clone
<220>
   <221> misc_feature
   <222> (14)..(16)
   <223> Deletion of 38 nucleotides + addition of 1 nucleotide
<400> 54
   gacccaagct ggctccccta tctagatatg aaa 33
<210> 55
   <211> 43
   <212> DNA
   <213> Artificial
<220>
   <223> mutated fragment of a clone
<400> 55
   tctagagcaa cacggaagga attatcccta tctagatatg aaa 43
<210> 56
   <211> 42
   <212> DNA
   <213> Artificial
<220>
   <223> mutated fragment of a clone
<400> 56
   tctagagcaa cacggaagga attaccctat ctagatatga aa 42
<210> 57
   <211> 41
   <212> DNA
   <213> Artificial
<220>
   <223> mutated fragment of a clone
<400> 57
   tctagagcaa cacggaagga attacctatc tagatatgaa a 41
<210> 58
   <211> 42
   <212> DNA
   <213> Artificial
<220>
   <223> mutated fragment of a clone
<400> 58
   tctagagcaa cacggaagga attaccctgt ctagatatga aa 42
<210> 59
   <211> 20
   <212> DNA
   <213> Artificial
<220>
   <223> mutated fragment of a clone
<220>
   <221> misc_feature
   <222> (16)..(17)
   <223> Deletion of 24 nucleotides
<400> 59
   caacacggaa ggaattgaaa 20
<210> 60
   <211> 36
   <212> DNA
   <213> Artificial
<220>
   <223> mutated fragment of a clone
<220>
   <221> misc_feature
   <222> (21)..(22)
   <223> Deletion of 29 nucleotides
<400> 60
   tctagagcaa cacggaagga aatcacgcca tgtagt 36
<210> 61
   <211> 37
   <212> DNA
   <213> Artificial
<220>
   <223> mutated fragment of a clone
<220>
   <221> misc_feature
   <222> (25)..(26)
   <223> Deletion of 30 nucleotides
<400> 61
   tctagagcaa cacggaagga attacgccat gtagtgt 37
<210> 62
   <211> 37
   <212> DNA
   <213> Artificial
<220>
   <223> mutated fragment of a clone
<220>
   <221> misc_feature
   <222> (18)..(19)
   <223> Deletion of 41 nucleotides
<400> 62
   gctagcgctc tagagcaatc acgccatgta gtgtatt 37
<210> 63
   <211> 28
   <212> DNA
   <213> Artificial
<220>
   <223> mutated fragment of a clone
<220>
   <221> misc_feature
   <222> (9)..(10)
   <223> Deletion of 97 nucleotides
<400> 63
   caacacggat tcgaattcga gctcgccc 28
<210> 64
   <211> 33
   <212> DNA
   <213> Artificial
<220>
   <223> mutated fragment of a clone
<220>
   <221> misc_feature
   <222> (14)..(15)
   <223> Deletion of 115 nucleotides
<400> 64
   tctagagcaa cacgcccggg gatcctctag agt 33
<210> 65
   <211> 43
   <212> DNA
   <213> Artificial
<220>
   <223> mutated fragment of a clone
<220>
   <221> misc_feature
   <222> (30)..(31)
   <223> Deletion of 143 nucleotides
<400> 65
   tctagagcaa cacggaagga attaccctgt gcaagaagca gat 43
<210> 66
   <211> 43
   <212> DNA
   <213> Artificial
<220>
   <223> mutated fragment of a clone
<220>
   <221> misc_feature
   <222> (23)..(25)
   <223> Addition of 1 nucleotide
<400> 66
   tctagagcaa cacggaagga attttcccta tctagatatg aaa 43
<210> 67
   <211> 33
   <212> DNA
   <213> Artificial
<220>
   <223> mutated fragment of a clone
<220>
   <221> misc_feature
   <222> (26)..(29)
   <223> Addition of 2 nucleotides
<400> 67
   tctagagcaa cacggaagga attaccattg aaa 33
<210> 68
   <211> 30
   <212> DNA
   <213> Artificial
<220>
   <223> mutated fragment of a clone
<220>
   <221> misc_feature
   <222> (15)..(18)
   <223> Deletion of 24 nucleotides + addition of 2 nucleotides
<400> 68
   caacacggaa ggaatagtct agatatgaaa 30
<210> 69
   <211> 38
   <212> DNA
   <213> Artificial
<220>
   <223> mutated fragment of a clone
<220>
   <221> misc_feature
   <222> (20)..(27)
   <223> Deletion of 90 nucleotides + addition of 6 nucleotides
<400> 69
   tctagagcaa cacggaagga catggtccat tcgaattc 38
<210> 70
   <211> 43
   <212> DNA
   <213> Artificial
<220>
   <223> mutated fragment of a clone
<400> 70
   tctagagcaa cacggaagga attatcccta tctagatatg aaa 43
<210> 71
   <211> 42
   <212> DNA
   <213> Artificial
<220>
   <223> mutated fragment of a clone
-<400> 71
tctagagcaa cacggaagga attaccctgt ctagatatga aa 42
<210> 72
   <211> 42
   <212> DNA
   <213> Artificial
<220>
   <223> mutated fragment of a clone
<400> 72
   tctagagcaa cacggaagga attaccctat ctagatatga aa 42
<210> 73
   <211> 29
   <212> DNA
   <213> Artificial
<220>
   <223> mutated fragment of a clone
<220>
   <221> misc_feature
   <222> (14)..(15)
   <223> Deletion of 23 nucleotides
<400> 73
   ctctagagca acactatcta gatatgaaa 29
<210> 74
   <211> 24
   <212> DNA
   <213> Artificial
<220>
   <223> mutated fragment of a clone
<220>
   <221> misc_feature
   <222> (9)..(10)
   <223> Deletion of 27 nucleotides
<400> 74
   tctagagcat atctagatat gaaa 24
<210> 75
   <211> 24
   <212> DNA
   <213> Artificial
<220>
   <223> mutated fragment of a clone
<220>
   <221> misc_feature
   <222> (17)..(18)
   <223> Deletion of 28 nucleotides
<400> 75
   ctctagagca acacggatat gaaa 24
<210> 76
   <211> 21
   <212> DNA
   <213> Artificial
<220>
   <223> mutated fragment of a clone
<220>
   <221> misc_feature
   <222> (18)..(19)
   <223> Deletion of 30 nucleotides
<400> 76
   tctagagcaa cacggaagaa a 21
<210> 77
   <211> 37
   <212> DNA
   <213> Artificial
<220>
   <223> mutated fragment of a clone
<220>
   <221> misc_feature
   <222> (22)..(23)
   <223> Deletion of 30 nucleotides
<400> 77
   tctagagcaa cacggaagga atcacgccat gtagtgt 37
<210> 78
   <211> 15
   <212> DNA
   <213> Artificial
<220>
   <223> mutated fragment of a clone
<220>
   <221> misc_feature
   <222> (7)..(8)
   <223> Deletion of 36 nucleotides
<400> 78
   tctagagata tgaaa 15
<210> 79
   <211> 40
   <212> DNA
   <213> Artificial
<220>
   <223> mutated fragment of a clone
<220>
   <221> misc_feature
   <222> (14)..(15)
   <223> Deletion of 42 nucleotides
<400> 79
   tctagagcaa cacgccatgt agtgtattga ccgattcctt 40
<210> 80
   <211> 31
   <212> DNA
   <213> Artificial
<220>
   <223> mutated fragment of a clone
<220>
   <221> misc_feature
   <222> (8)..(9)
   <223> Deletion of 49 nucleotides
<400> 80
   tctagagcca tgtagtgtat tgaccgattc c 31
<210> 81
   <211> 34
   <212> DNA
   <213> Artificial
<220>
   <223> mutated fragment of a clone
<220>
   <221> misc_feature
   <222> (14)..(15)
   <223> Deletion of 92 nucleotides
<400> 81
   tggcttatcg aaatcacgcc atgtagtgta ttga 34
<210> 82
   <211> 36
   <212> DNA
   <213> Artificial
<220>
   <223> mutated fragment of a clone
<220>
   <221> misc_feature
   <222> (20)..(21)
   <223> Deletion of 217 nucleotides
<400> 82
   tctagagcaa cacggaagga gaccaccatg tgccga 36
<210> 83
   <211> 71
   <212> DNA
   <213> Artificial
<220>
   <223> mutated fragment of a clone
<220>
   <221> misc_feature
   <222> (16)..(63)
   <223> Deletion of 26 nucleotides + Insertion of 46 nucleotides
<400> 83
   tctagagcaa cacggagcca cccagcacgc aagccaggaa cactgtctgg ttcacccctc 60
   tggatatgaa a 71
<210> 84
   <211> 128
   <212> DNA
   <213> Artificial
<220>
   <223> mutated fragment of a clone
<220>
   <221> misc_feature
   <222> (14)..(116)
   <223> Deletion of 19 nucleotides + Insertion of 101 nucleotides
<400> 84
   tctagagcaa cacgcccggg gatctcgagg tcaccctgac ggtgtcgtcc atcacagttt 60
   gccagtgata cacatgggga tcagcaatcg cgcataatcg atattaccct gttatcccta 120
   tctagata 128
<210> 85
   <211> 50
   <212> DNA
   <213> Artificial
<220>
   <223> mutated fragment of a clone
<400> 85
   tctagagcaa cacggaagga attaccctgt atccctatct agatatgaaa 50
<210> 86
   <211> 49
   <212> DNA
   <213> Artificial
<220>
   <223> mutated fragment of a clone
<400> 86
   tctagagcaa cacggaagga attaccctgt tccctatcta gatatgaaa 49
<210> 87
   <211> 45
   <212> DNA
   <213> Artificial
<220>
   <223> mutated fragment of a clone
<400> 87
   tctagagcaa cacggaagga attaccctgt tatctagata tgaaa 45
<210> 88
   <211> 44
   <212> DNA
   <213> Artificial
<220>
   <223> mutated fragment of a clone
<400> 88
   tctagagcaa cacggaagga atttatccct atctagatat gaaa 44
<210> 89
   <211> 43
   <212> DNA
   <213> Artificial
<220>
   <223> mutated fragment of a clone
<400> 89
   tctagagcaa cacggaagga attaccccta tctagatatg aaa 43
<210> 90
   <211> 42
   <212> DNA
   <213> Artificial
<220>
   <223> mutated fragment of a clone
<400> 90
   tctagagcaa cacggaagga attaccctgt ctagatatga aa 42
<210> 91
   <211> 41
   <212> DNA
   <213> Artificial
<220>
   <223> mutated fragment of a clone
<400> 91
   tctagagcaa cacggaagga attccctatc tagatatgaa a 41
<210> 92
   <211> 40
   <212> DNA
   <213> Artificial
<220>
   <223> mutated fragment of a clone
<400> 92
   tctagagcaa cacggaagga atccctatct agatatgaaa 40
<210> 93
   <211> 39
   <212> DNA
   <213> Artificial
<220>
   <223> mutated fragment of a clone
<400> 93
   tctagagcaa cacggaagga attaccctgt tatatgaaa 39
<210> 94
   <211> 38
   <212> DNA
   <213> Artificial
<220>
   <223> mutated fragment of a clone
<220>
   <221> misc_feature
   <222> (17)..(18)
   <223> Deletion of 33 nucleotides
<400> 94
   tctagagcaa cacggaaatc acgcccatgt agtgtatt 38
<210> 95
   <211> 111
   <212> DNA
   <213> Artificial
<220>
   <223> mutated fragment of a clone
<220>
   <221> misc_feature
   <222> (25)..(94)
   <223> Insertion of 68 nucleotides
<400> 95
   tctagagcaa cacggaagga attacgtcaa tagggggcgt actatgggaa catacgtttt     60
   ctcacataca gatgcctact ggtgtttttg atgccctatc tagatatgaa a             111
<210> 96
   <211> 160
   <212> DNA
   <213> Artificial
<220>
   <223> mutated fragment of a clone
<220>
   <221> misc_feature
   <222> (29)..(146)
   <223> Insertion of 116 nucleotides
<400> 96
   tctagagcaa cacggaagga attaccctgc tcaacagcgg taagatcctt gagagttttc     60
   gccccgaaga acgttttcca atgatgagca cttttaaagt tctgctatgt ggcgcggtat    120
   tatcccgtat tgacgccggg caagatatct agatatgaaa                          160
<210> 97
   <211> 153
   <212> DNA
   <213> Artificial
<220>
   <223> mutated fragment of a clone
<220>
   <221> misc_feature
   <222> (33)..(136)
   <223> Insertion of 102 nucleotides
<400> 97
   tctagagcaa cacggaagga attaccctgt tatcctgtta tgtttttcca taggctccgc 60
   ccccctgacg agcatcacaa aaatcgacgc tcaagtcaga ggtggcgaaa cccgacagga 120
   ctataaagat accatcccta tctagatatg aaa 153
<210> 98
   <211> 48
   <212> DNA
   <213> Artificial
<220>
   <223> mutated fragment of a clone
<400> 98
   tctagagcaa cacggaagga attaccttat ccctatctag atatgaaa 48
<210> 99
   <211> 42
   <212> DNA
   <213> Artificial
<220>
   <223> mutated fragment of a clone
<400> 99
   tctagagcaa cacggaagga attaccctat ctagatatga aa 42
<210> 100
   <211> 52
   <212> DNA
   <213> Artificial
<220>
   <223> mutated fragment of a clone
<220>
   <221> misc_feature
   <222> (31)..(33)
   <223> Addition of 1 nucleotide
<400> 100
   tctagagcaa cacggaagga attaccctgt ttatccctat ctagatatga aa 52
<210> 101
   <211> 53
   <212> DNA
   <213> Artificial
<220>
   <223> mutated fragment of a clone
<220>
   <221> misc_feature
   <222> (30)..(33)
   <223> Addition of 2 nucleotides
<400> 101
   tctagagcaa cacggaagga attaccctgt cttatcccta tctagatatg aaa 53
<210> 102
   <211> 54
   <212> DNA
   <213> Artificial
<220>
   <223> mutated fragment of a clone
<220>
   <221> misc_feature
   <222> (33)..(38)
   <223> Addition of 4 nucleotides
<400> 102
   tctagagcaa cacggaagga attaccctgt tattgggcct atctagatat gaaa 54
<210> 103
   <211> 55
   <212> DNA
   <213> Artificial
<220>
   <223> mutated fragment of a clone
<220>
   <221> misc_feature
   <222> (31)..(36)
   <223> Addition of 4 nucleotides
<400> 103
   tctagagcaa cacggaagga attaccctgt tggacatccc tatctagata tgaaa 55
<210> 104
   <211> 53
   <212> DNA
   <213> Artificial
<220>
   <223> mutated fragment of a clone
<220>
   <221> misc_feature
   <222> (31)..(36)
   <223> Addition of 4 nucleotides
<400> 104
   tctagagcaa cacggaagga attaccctgt ttgctcccta tctagatatg aaa 53
<210> 105
   <211> 52
   <212> DNA
   <213> Artificial
<220>
   <223> mutated fragment of a clone
<220>
   <221> misc_feature
   <222> (28)..(33)
   <223> Addition of 4 nucleotides
<400> 105
   tctagagcaa cacggaagga attaccctcc cgatccctat ctagatatga aa 52
<210> 106
   <211> 55
   <212> DNA
   <213> Artificial
<220>
   <223> mutated fragment of a clone
<220>
   <221> misc_feature
   <222> (29)..(35)
   <223> Addition of 5 nucleotides
<400> 106
   tctagagcaa cacggaagga attaccctgc ctcatatccc tatctagata tgaaa 55
<210> 107
   <211> 38
   <212> DNA
   <213> Artificial
<220>
   <223> mutated fragment of a clone
<220>
   <221> misc_feature
   <222> (21)..(27)
   <223> Addition of 5 nucleotides
<400> 107
   tctagagcaa cacggaagga acccccctag atatgaaa 38
<210> 108
   <211> 57
   <212> DNA
   <213> Artificial
<220>
   <223> mutated fragment of a clone
<220>
   <221> misc_feature
   <222> (31)..(38)
   <223> Addition of 6 nucleotides
<400> 108
   tctagagcaa cacggaagga attaccctgt tcctcttatc cctatctaga tatgaaa 57
<210> 109
   <211> 53
   <212> DNA
   <213> Artificial
<220>
   <223> mutated fragment of a clone
<220>
   <221> misc_feature
   <222> (33)..(36)
   <223> Addition of 2 nucleotides
<400> 109
   tctagagcaa cacggaagga attaccctgt tatgacccta tctagatatg aaa 53
<210> 110
   <211> 508
   <212> PRT
   <213> Homo sapiens
<220>
   <221> MISC_FEATURE
   <222> (1)..(508)
   <223> TdT (Terminal deoxynucleotidyl - terminal transferase)
<400> 110
<210> 111
   <211> 16
   <212> DNA
   <213> Artificial
<220>
   <223> Fragment of vector pCMV-H2Kd-CD8-CD4
<400> 111
   taccctgtta tcccta 16
<210> 112
   <211> 16
   <212> DNA
   <213> Artificial
<220>
   <223> Mutated fragment of cleaved vector pCMV-H2Kd-CD8-CD4
<220>
   <221> misc_feature
   <222> (12)..(16)
   <223> N = A, C, T or G or any modified nucleotide
<400> 112
   taccctgtta tnnnnn 16
<210> 113
   <211> 27
   <212> DNA
   <213> Artificial
<220>
   <223> Mutated repaired junction
<220>
   <221> misc_feature
   <222> (12)..(16)
   <223> N = A, C, T or G or any modified nucleotide
<220>
   <221> misc_feature
   <222> (17)..(18)
   <223> Gap filling with N = A, T, C or G or any modified nucleotide
<400> 113
   taccctgtta tnnnnnnntt atcccta 27